# EUROPEAN PATENT APPLICATION

(11) **EP 1 757 281 A2**
(43) Date of publication of application: **28.02.2007**
(21) Application number: 06124055.2
(22) Date of filing: 04.02.2003
(51) Int. Cl.: A61K 9/72, A61K 31/18, A61K 31/58, A61P 11/06

(54) **Formulation for inhalation comprising a glucocorticoid and a beta 2-adrenoreceptor agonist**

(30) Priority: 04.02.2002 US 66961
(62) Divisional of application: 03702732.3
(71) Applicant: GLAXO GROUP LIMITED, Greenford, Middlesex UB6 ONN (GB)
(72) Inventor: Biggadike, Keith, Stevenage, Hertfordshire SG1 2NY (GB); Jones, Paul, Stevenage, Hertfordshire SG1 2NY (GB); Payne, Jeremy, John, Stevenage, Hertfordshire SG1 2NY (GB)
(74) Representative: Pritchard, Judith

(57) **Abstract**

According to one aspect of the invention, there is provided a pharmaceutical formulation for administration by inhalation comprising a compound of formula (I), 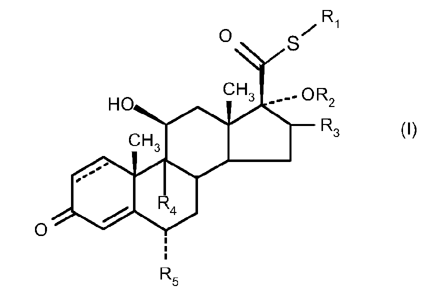 wherein
R₁ represents C₁₋₆ alkyl or C₁₋₆ haloalkyl;
R₂ represents -C(=O)-aryl or -C(=O)-heteroaryl;
R₃ represents hydrogen, methyl (which may be in either the α or β configuration) or methylene;
R₄ and R₅ are the same or different and each represents hydrogen or halogen; and
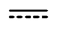 represents a single or a double bond;
and salts and solvates thereof together with a long-acting β₂-adrenoreceptor agonist which formulation has a therapeutically useful effect in the treatment of inflammatory disorders of the respiratory tract over a period of 24 hours or more.

## Description

This application is a Continuation-in-part of International Patent Application Serial No. PCT.GB01.03499 filed 3 August 2001, based upon United Kingdom Patent Application No. GB0108800.4 filed 7 April 2001.

The present invention relates to novel formulations of anti-inflammatory and anti-allergic compounds of the androstane series and β₂-adrenoreceptor agonists and to processes for their preparation. The present invention also relates to pharmaceutical formulations containing the compounds and to therapeutic uses thereof, particularly for the treatment of inflammatory and allergic conditions.

Glucocorticoids which have anti-inflammatory properties are known and are widely used for the treatment of inflammatory disorders or diseases such as asthma and rhinitis. For example, US Patent 4335121 discloses 6α, 9α-Difluoro-17α-(1-oxopropoxy)-11β-hydroxy-16α-methyl-3-oxo-androsta-1,4-diene-17β-carbothioic acid *S*-fluoromethyl ester (known by the generic name of fluticasone propionate) and derivatives thereof. The use of glucocorticoids generally, and especially in children, has been limited in some quarters by concerns over potential side effects. The side effects that are feared with glucocorticoids include suppression of the Hypothalamic-Pituitary-Adrenal (HPA) axis, effects on bone growth in children and on bone density in the elderly, ocular complications (cataract formation and glaucoma) and skin atrophy. Certain glucocorticoid compounds also have complex paths of metabolism wherein the production of active metabolites may make the pharmacodynamics and pharmacokinetics of such compounds difficult to understand. Whilst the modern steroids are very much safer than those originally introduced it remains an object of research to produce new molecules which have excellent anti-inflammatory properties, with predictable pharmacokinetic and pharmacodynamic properties, with an attractive side effect profile, and with a convenient treatment regime.

In our application PCT.GB.0103.499 we have identified a novel series of glucocorticoids, which substantially meets these objectives.

Current combination products comprising glucocorticoids and β₂-adrenoreceptor receptor agonists are normally administered on a twice daily regime.

It is considered that once-per-day dosing is significantly more convenient to patients than a twice-per-day dosing regime, which is likely to lead to greater patient compliance and hence better disease control and a product providing such a dosing regime has been sought.

It is an objective of the present invention to provide safe and effective once-per-day combination formulation.

According to a first aspect of the invention, there is provided a pharmaceutical formulation for administration by inhalation comprising a compound of formula (I), 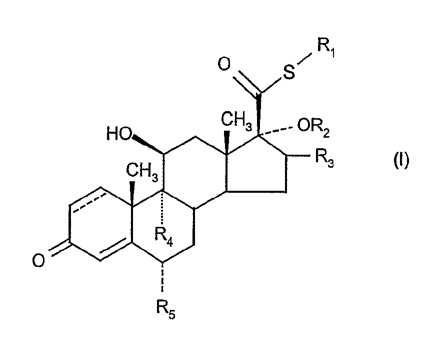 wherein
R₁ represents C₁₋₆ alkyl or C₁₋₆ haloalkyl;
R₂ represents -C(=O)-aryl or -C(=O)-heteroaryl;
R₃ represents hydrogen, methyl (which may be in either the α or β configuration) or methylene;
R₄ and R₅ are the same or different and each represents hydrogen or halogen; and
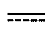 represents a single or a double bond;
or a salt or solvate thereof, together with a long-acting β₂-adrenoreceptor agonist which formulation has a therapeutically useful effect in the treatment of inflammatory disorders of the respiratory tract over a period of 24 hours or more.

References to the term "aryl" include references to phenyl which may be optionally substituted with one or more substituents.

References to the term "heteroaryl" include references to 5 or 6 membered heterocyclic aromatic rings containing 1-3 hetero atoms selected from N, O and S (e.g. pyridinyl, pyrimidinyl, thienyl (eg thien-2-yl or thien-3-yl), furanyl (eg furan-2-yl or furan-3-y), pyrrolyl (eg 1H-pyrrol-2-yl), oxazolyl, thiadiazolyl (eg 1,2,3-thiadiazol-5-yl, 1,2,5-thiadiazol-3-yl or 1,2,3-thiadiazol-4-yl)). In one respect the heterocycle is preferably thiophenyl, pyrrolyl or furanyl, more preferably thiophenyl or furanyl. Further examples include thiazolyl (eg 1,3-thiazolyl-5-yl or 1,3-thiazolyl-4-yl), isoxazolyl (eg isoxazol-5-yl or isoxazol-4-yl), isothiazolyl (eg isothiazol-3-yl or isothiazol-5-yl), pyrazolyl (eg 1H-pyrazol-5-yl) and imidazolyl (eg 1H-imidazol-5-yl).

All of the previously named heterocycles may be optionally substituted with one or more (e.g. 1 or 2) substituents.

Examples of substituents for aryl and heteroaryl include C₁₋₆ alkyl (e.g. methyl) or halogen (e.g. chlorine or bromine). Other examples include C₁₋₆ alkoxy (e.g. methoxy or ethoxy).

Examples of substituted furanyl include 3-Me-furan-2-yl, 5-Br-furan-2-yl, 2-Me-furan-3-yl and 2,5-diMe-furan-3-yl. Examples of substituted thienyl include 3-Me-thien-2-yl, 5-Me-thien-2-yl, 5-Cl-thien-2-yl, 3-Cl-thien-2-yl, 3-Br-thien-2-yl, 3-Ethoxy-thien-2-yl, 4-methoxy-thien-3-yl, 2,5-diCl-thien-3-yl and 4-methoxy-5-Cl-thien-3-yl. Examples of substituted pyrrolyl include 1-Me-1H-pyrrol-2-yl. Examples of substituted thiazolyl include 4-Me-1,3-thiazol-5-yl, 2,4-diMe-1,3-thiazol-5-yl and 2-Me-1,3-thiazol-4-yl. Examples of substituted thiadiazolyl include 4-Me-1,2,3-thiadiazol-5-yl. Examples of substituted isoxazolyl include 3-Me-isoxazol-5-yl, 5-Me-isoxazol-3-yl, 5-Me-isoxazol-4-yl and 3,5-diMe-isoxazol-4-yl. Examples of substituted pyrazolyl include 1,3-diMe-1 H-pyrazol-5-yl and 1-Et-3-Me-1H-pyrazol-5-yl. Examples of substituted imidazolyl include 1-Me-1H-imidazol-5-yl.

Examples of solvates include hydrates.

Examples of salts of compounds of formula (I) include physiologically acceptable salts which may be formed with basic compounds (such as when heteroaryl is basic) eg. acetate, benzoate, citrate, succinate, lactate, tartrate, fumarate and maleate.

References hereinafter to a compound of formula (I) include both compounds of formula (I) and salts and solvates thereof, particularly pharmaceutically acceptable salts and solvates.

It will be appreciated that the invention includes within its scope compositions comprising all stereoisomers of the compounds of formula (I) and mixtures thereof.
Preferably, the absolute stereochemistry will be as shown in the representation of compounds of formula (I).

Examples of C₁₋₆ haloalkyl that R₁ may represent include C₁₋₆ alkyl substituted by 1-3 halogen atoms, preferably 1 halogen atom. Preferred halogen atoms are selected from bromine, chlorine and fluorine. Examples of C₁₋₆alkyl that R₁ may represent include methyl.

We prefer R₁ to represent fluoromethyl, chloromethyl, bromomethyl or 2'-fluoroethyl, especially fluoromethyl.

We prefer R₂ to represent -C(=O)-heteroaryl. Preferably the heteroaryl is a 5-membered heterocyclic aromatic ring containing 1 to 3 heteroatoms selected from O, N and S which may optionally be substituted. In one respect, preferably heteroaryl represents furanyl, pyrrolyl or thiophenyl, more preferably furanyl or thiophenyl eg 2-furanyl, 3-furanyl, 2-thiophenyl or 3-thiophenyl, especially furanyl, particularly 2-furanyl. However it may also be preferred that heteroaryl represents pyrrolyl or thiophenyl, more preferably thiophenyl eg 2-thiophenyl or 3-thiophenyl which pyrrolyl or thiophenyl may be optionally substituted.

Of particular interest are compounds in which the heteroaryl is a 5-membered heterocyclic aromatic ring containing 2 heteroatoms selected from O, N and S.

Thus a further set of preferred compounds is that in which R₂ represents optionally substituted thiazolyl. A further set of preferred compounds is that in which R₂ represents optionally substituted isothiazolyl. A further set of preferred compounds is that in which R₂ represents optionally substituted pyrazolyl. A further set of preferred compounds is that in which R₂ represents optionally substituted isoxazolyl. A further set of preferred compounds is that in which R₂ represents optionally substituted isothiazolyl. A further set of preferred compounds is that in which R₂ represents optionally substituted imidazolyl.

Of particular interest is compounds in which the heteraryl is a 5-membered heterocyclic aromatic ring containing 3 heteroatoms selected from O, N and S.

Thus a further set of preferred compounds is that in which R₂ represents optionally substituted thiadiazolyl.

Compounds in which R₂ represents -C(=O)-aryl (preferably aryl represents phenyl) are also of particular interest.

We prefer R₃ to represent methyl, especially methyl in the α configuration. Compounds in which R₃ represents methyl in the β configuration are also of particular interest.

Compounds of formula (I) in which R₄ and R₅, which can be the same or different, each represents hydrogen, fluorine or chlorine, particularly hydrogen or fluorine, are preferred. Especially preferred are compounds in which both R₄ and R₅ are fluorine.

Preferably, 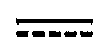 represents a double bond. Compounds in which 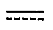 represents a single bond are also of particular interest.

A particularly preferred group of compounds of formula (I) are those in which R₁ is fluoromethyl; R₂ is -C(=O)-2-furanyl; R₃ is methyl; R₄ and R₅, which can be the same or different, each represents hydrogen or fluorine, especially fluorine, and 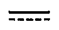 represents a single or a double bond.

It is to be understood that the present invention covers formulations comprising all combinations of particularly and preferred groups for formula (I) referred to hereinabove.

Preferred compounds of formula (I) include:
6α,9α-Difluoro-17α-[(2-furanylcarbonyl)oxy]-11β-hydroxy-16α-methyl-3-oxo-androsta-1,4-diene-17β-carbothioic acid *S*-fluoromethyl ester;
6α,9α-Difluoro-17α-[(3-furanylcarbonyl)oxy]-11β-hydroxy-16α-methyl-3-oxo-androsta-1,4-diene-17β-carbothioic acid *S*-fluoromethyl ester;
6α,9α-Difluoro-11β-hydroxy-16α-methyl-3-oxo-17α-[(2-thienylcarbonyl)oxy]-androsta-1,4-diene-17β-carbothioic acid *S*-fluoromethyl ester;
6α,9α-Difluoro-11β-hydroxy-16α-methyl-3-oxo-17α-[(3-thienylcarbonyl)oxy]-androsta-1,4-diene-17β-carbothioic acid *S*-fluoromethyl ester;
17α-(Benzoyl)oxy-6α,9α-Difluoro-11β-hydroxy-16α-methyl-3-oxo-androsta-1,4-diene-17β-carbothioic acid *S*-fluoromethyl ester;
9α-Fluoro-17α-[(2-furanylcarbonyl)oxy]-11β-hydroxy-16β-methyl-3-oxo-androsta-1,4-diene-17β-carbothioic acid *S*-fluoromethyl ester; and
6α,9α-Difluoro-17α-[(2-furanylcarbonyl)oxy]-11β-hydroxy-16α-methyl-3-oxo-androst-4-ene-17β-carbothioic acid *S*-fluoromethyl ester
or a salt or solvate thereof.

Particularly preferred compounds of formula (I) include:
6α,9α-Difluoro-17α-[(2-furanylcarbonyl)oxy]-11β-hydroxy-16α-methyl-3-oxo-androsta-1,4-diene-17β-carbothioic acid *S*-fluoromethyl ester;
6α,9α-Difluoro-17α-[(3-furanylcarbonyl)oxy]-11β-hydroxy-16α-methyl-3-oxo androsta-1,4-diene-17β-carbothioic acid *S*-fluoromethyl ester;
6α,9α-Difluoro-11β-hydroxy-16α-methyl-3-oxo-17α-[(2-thienylcarbonyl)oxy]-androsta-1,4-diene-17β-carbothioic acid *S*-fluoromethyl ester;
6α,9α-Difluoro-11β-hydroxy-16α-methyl-3-oxo-17α-[(3-thienylcarbonyl)oxy]-androsta-1,4-diene-17β-carbothioic acid *S*-fluoromethyl ester;
6α,9α-Difluoro-17α-[(2-furanylcarbonyl)oxy]-11β-hydroxy-16α-methyl-3-oxo-androst-4-ene-17β-carbothioic acid *S*-fluoromethyl ester
or a salt or solvate thereof.

A highly preferred compound of formula (I) is 6α,9α-Difluoro-11β-hydroxy-16α-methyl-17α-[(4-methyl-1,3-thiazole-5-carbonyl)oxy]-3-oxo-androsta-1,4-diene-17β-carbothioic acid *S*-fluoromethyl ester or a salt or solvate thereof. (Example 41)

The compounds named as Examples 8-43 are also of particular interest, especially Examples 8-22 and 24-43, most especially examples 8-9, 11-13, 15-22 and 24-43.

In some embodiments, preferred long-acting β₂-adrenoreceptor agonists include salmeterol or formoterol.

Particularly preferred long-acting β₂-adrenoreceptor agonists have a therapeutic bronchodilatory effect over a 24 hour period.

In some embodiments, preferred long acting β₂-adrenoreceptor agonists include those described in WO 02066422, WO02070490 and WO02076933.

Especially preferred long-acting β₂-adrenoreceptor agonists include compounds of formula(M): 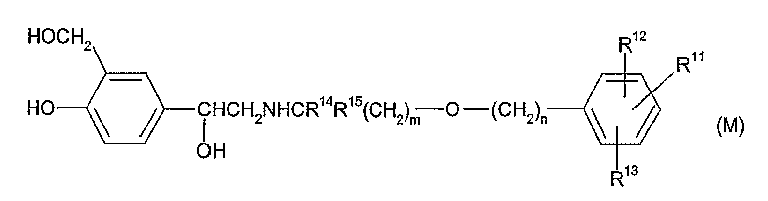 or a salt or solvate thereof, wherein:
m is an integer of from 2 to 8;
n is an integer of from 3 to 11,
with the proviso that m + n is 5 to 19,
R¹¹ is -XSO₂NR¹⁶R¹⁷ wherein X is -(CH₂)ₚ- or C₂₋₆ alkenylene;
R¹⁶ and R¹⁷ are independently selected from hydrogen, C₁₋₆alkyl, C₃₋₇cycloalkyl, C(O)NR¹⁸R¹⁹, phenyl, and phenyl (C₁₋₄alkyl)-,
or R¹⁶ and R¹⁷, together with the nitrogen to which they are bonded, form a 5-, 6-, or 7- membered nitrogen containing ring, and R¹⁶ and R¹⁷ are each optionally substituted by one or two groups selected from halo, C₁₋₆alkyl, C₁₋₆haloalkyl, C₁₋₆alkoxy, hydroxy-substituted C₁₋₆alkoxy, -CO₂R¹⁸, -SO₂NR¹⁸R¹⁹, -CONR¹⁸R¹⁹, -NR¹⁸C(O)R¹⁹, or a 5-, 6- or 7-membered heterocylic ring;
R¹⁸ and R¹⁹ are independently selected from hydrogen, C₁₋₆alkyl, C₃₋₆cycloalkyl, phenyl, and phenyl (C₁₋₄alkyl)-; and
p is an integer of from 0 to 6, preferably from 0 to 4;
R¹² and R¹³ are independently selected from hydrogen, C₁₋₆alkyl, C₁₋₆alkoxy, halo, phenyl, and C₁₋₆haloalkyl; and
R¹⁴ and R¹⁵ are independently selected from hydrogen and C₁₋₄alkyl with the proviso that the total number of carbon atoms in R¹⁴ and R¹⁵ is not more than 4.

The compounds of formula (I) have potentially beneficial anti-inflammatory or anti-allergic effects, particularly upon topical administration, demonstrated by, for example, their ability to bind to the glucocorticoid receptor and to illicit a response via that receptor with long lasting effect. Hence, the compounds of formula (I) are useful in the treatment of inflammatory and/or allergic disorders, especially in once-per-day therapy.

Preferably in the pharmaceutical formulation of the invention the compound of formula (I) or a solvate thereof and the long-acting β₂-adrenoreceptor agonist are both present in particulate form.

The pharmaceutical formulation of the invention may comprise one or more excipients.

By the term "excipient", as used herein, it is meant to mean substantially inert materials that are nontoxic and do not interact with other components of a composition in a deleterious manner including, but not limited to, pharmaceutical grades of: carbohydrates, organic and inorganic salts, polymers, amino acids, phospholipids, wetting agents, emulsifiers, surfactants, poloxamers, pluronics, and ion exchange resins, and combinations thereof, a non-exhaustive list of examples of which are provided below:
Carbohydrates, including: monosaccharides, such as, but not limited to, fructose ; disaccharides, such as, but not limited to lactose, and combinations and derivatives thereof; polysaccharides, such as, but not limited to, cellulose and combinations and derivatives thereof; oligosaccharides, such as, but not limited to, dextrins, and combinations and derivatives thereof; polyols, such as but not limited to sorbitol, and combinations and derivatives thereof;
Organic and inorganic salts, including but not limited to sodium or calcium phosphates, magnesium stearate, and combinations and derivatives thereof;
Polymers, including: natural biodegradable protein polymers including, but not limited to, gelatin and combinations and derivatives thereof; Natural biodegradable polysaccharide polymers including, but not limited to, chitin and starch, crosslinked starch, and combinations and derivatives thereof; Semisynthetic biodegradable polymers including, but not limited to, derivatives of chitosan; Synthetic biodegradable polymers including but not limited to polyethylene glycols (PEG), polylactic acid (PLA), synthetic polymers including but not limited to polyvinyl alcohol and combinations and derivatives thereof;
Amino acids including but not limited to including non-polar amino acids, such as leucine and combinations and derivatives thereof;
Phospholipids, including lecithins and combinations and derivatives thereof;
Wetting agents/ Surfactants/Emulsifiers, including, but not limited to gum acacia, cholesterol, fatty acids including, combinations and derivatives thereof;
Poloxamers/ Pluronics: including but not limited to poloxamer 188, Pluronic® F-108, and combinations and derivations thereof;
Ion exchange resins: including but not limited to amberlite IR120 and combinations and derivatives thereof;
and combinations of the noted excipients.

The pharmaceutical formulation of the invention preferably comprises a particulate carrier, especially lactose.

The pharmaceutical formulation of the invention preferably further comprises a liquefied propellant gas.

The inflammatory disorder of the respiratory tract is preferably asthma.

According to a second aspect of the present invention there is provided a method of treatment of a inflammatory disorder of the respiratory tract once-per-day which comprises administration of a pharmaceutical formulation according to the first aspect of the present invention.

The inflammatory disorder of the respiratory tract is preferably asthma.

According to a third aspect of the present invention there is provided an inhaler containing a plurality of doses of a pharmaceutical formulation comprising a compound of formula (I) 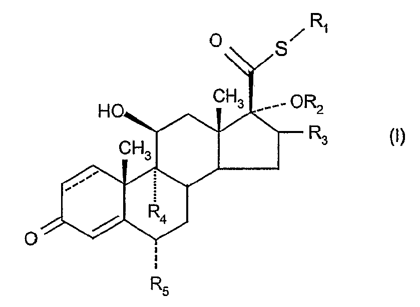 wherein
R₁ represents C₁₋₆ alkyl or C₁₋₆ haloalkyl;
R₂ represents -C(=O)-aryl or -C(=O)-heteroaryl;
R₃ represents hydrogen, methyl (which may be in either the α or β configuration) or methylene;
R₄ and R₅ are the same or different and each represents hydrogen or halogen; and
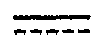 represents a single or a double bond;
or a salt or solvate thereof, together with a long-acting β₂-adrenoreceptor agonist which formulation has a therapeutically useful effect in the treatment of inflammatory disorders of the respiratory tract over a period of 24 hours or more, and which doses are suitable for once-per-day administration of the formulation by inhalation.

Preferably, in the inhaler, the compound of formula (I) or a salt or solvate thereof and the long-acting β₂-adrenoreceptor agonist are both present in particulate form.

The inhaler of the invention further comprises a particulate carrier, especially lactose.

The inhaler of the invention preferably further comprises a liquefied propellant gas.

Preferably the inhaler containing a plurality of doses of the pharmaceutical formulation of the invention wherein the compound of formula (I) or a salt orsolvate thereof and the long-acting β₂-adrenoreceptor agonists are both present in particulate form.

Preferably in the inflammatory disorder of the respiratory tract is asthma.

In the inhaler of the present invention the long-acting β₂-adrenoreceptor agonist is preferably a compound of formula (M): 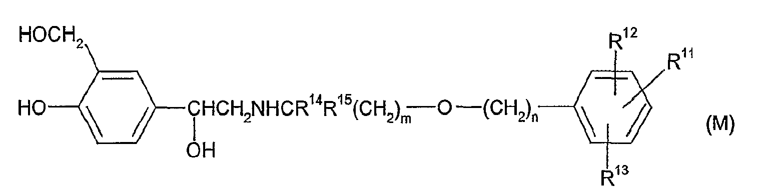 or a salt or solvate thereof, wherein:
m is an integer of from 2 to 8;
n is an integer of from 3 to 11,
with the proviso that m + n is 5 to 19,
R¹¹ is -XSO₂NR¹⁶R¹⁷ wherein X is -(CH₂)ₚ- or C₂₋₆ alkenylene;
R¹⁶ and R¹⁷ are independently selected from hydrogen, C₁₋₆alkyl, C₃₋₇cycloalkyl, C(O)NR¹⁸R¹⁹, phenyl, and phenyl (C₁₋₄alkyl)-,
or R¹⁶ and R¹⁷, together with the nitrogen to which they are bonded, form a 5-, 6-, or 7- membered nitrogen containing ring, and R¹⁶ and R¹⁷ are each optionally substituted by one or two groups selected from halo, C₁₋₆alkyl, C₁₋₆haloalkyl, C₁₋₆alkoxy, hydroxy-substituted C₁₋₆alkoxy, -CO₂R¹⁸, -SO₂NR¹⁸R¹⁹, -CONR¹⁸R¹⁹, -NR¹⁸C(O)R¹⁹, or a 5-, 6- or 7-membered heterocylic ring;
R¹⁸ and R¹⁹ are independently selected from hydrogen, C₁₋₆alkyl, C₃₋₆cycloalkyl, phenyl, and phenyl (C₁₋₄alkyl)-; and
p is an integer of from 0 to 6, preferably from 0 to 4;
R¹² and R¹³ are independently selected from hydrogen, C₁₋₆alkyl, C₁₋₆alkoxy, halo, phenyl, and C₁₋₆haloalkyl; and
R¹⁴ and R¹⁵ are independently selected from hydrogen and C₁₋₄alkyl with the proviso that the total number of carbon atoms in R¹⁴ and R¹⁵ is not more than 4.

In the inhaler of the invention the compound of formula (I) is preferably 6α,9α-Difluoro-11β-hydroxy-16α-methyl-17α-[(4-methyl-1,3-thiazole-5-carbonyl)oxy]-3-oxo-androsta-1,4-diene-17β-carbothioic acid *S*-fluoromethyl ester or a salt or solvate thereof.

According to another aspect of the present invention there is provided the use of a formulation according to the first aspect of the present invention for use in human or veterinary medicine in the treatment of patients with inflammatory and/or allergic condition, for treatment once per-day

According to another aspect of the present invention there is provided the use of a formulation as according to the first aspect of the present invention for the manufacture of a medicament for the treatment of patient with inflammatory and/or allergic conditions for treatment once-per-day.

There is also provided a method for the treatment of a human or animal subject with an anti-inflammatory and/or allergic condition, which method comprises administering to said human or animal subject an effective amount of formulation according to the first aspect, especially for administration once per day.

In the method of the invention the compound of formula (I) is preferably 6α,9α-Difluoro-11β-hydroxy-16α-methyl-17α-[(4-methyl-1,3-thiazole-5-carbonyl)oxy]-3-oxo-androsta-1,4-diene-17β-carbothioic acid *S*-fluoromethyl ester or a salt or solvate thereof.

Compounds of formula (I) are predicted to undergo highly efficient hepatic metabolism to yield the corresponding 17-β carboxylic acid (X) in which R₂-R₅ and 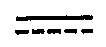 are as defined above as the sole major metabolite in rat and human *in vitro* systems. We have established that this is the case for Example 1 and metabolite (X) for Example 1 has been synthesised and demonstrated to be >1000 fold less active than the parent compound in *in vitro* functional glucocorticoid agonist assays. Analogues of (X) wherein R₂ represents a group other than 2-furanylcarbonyl are expected also to have very low activity.

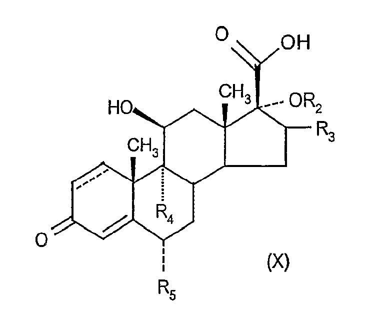

This efficient hepatic metabolism is reflected by *in vivo* data in the rat for certain examples, which have demonstrated plasma clearance at a rate approaching hepatic blood flow (Examples 1, 4, 19, 24, 25 and 28) and an oral bioavailability of <1%, consistent with extensive first-pass metabolism (Example 1).

*In vitro* metabolism studies in human hepatocytes have demonstrated that Example 1 is metabolised in an identical manner to fluticasone propionate but that conversion of Example 1 to the inactive acid metabolite occurs approximately 5-fold more rapidly than with fluticasone propionate. This very efficient hepatic inactivation would be expected to minimise systemic exposure in man leading to an improved safety profile.

Inhaled steroids are also absorbed through the lung and this route of absorption makes a significant contribution to systemic exposure. Reduced lung absorption could therefore provide an improved safety profile. Studies with Example 1 have shown significantly lower exposure to Example 1 than with fluticasone propionate after dry powder delivery to the lungs of anaesthetised pigs.

An improved safety profile is believed to allow the compounds of formula (I) to demonstrate the desired anti-inflammatory effects when administered once-per day. Once-per-day dosing is considered to be significantly more convenient to patients than the twice-per day dosing regime that is normally employed for fluticasone propionate.

*In vitro* lung tissue affinity experiments indicate significantly higher affinity of 6α,9α-Difluoro-11β-hydroxy-16α-methyl-17α-[(4-methyl-1,3-thiazole-5-carbonyl)oxy]-3-oxo-androsta-1,4-diene-17β-carbothioic acid *S*-fluoromethyl ester for human lung tissue preparations than does fluticasone proionate. This is supported also by cell transport and accumulation studies using human bronchial epithelial cells. These studies show reduced flux across the cell layer for 6α,9α-Difluoro-11β-hydroxy-16α-methyl-17α-[(4-methyl-1,3-thiazole-5-carbonyl)oxy]-3-oxo-androsta-1,4-diene-17β-carbothioic acid *S*-fluoromethyl ester compared with fluticasone propionate. The studies also show greater accumulation of the 6α,9α-Difluoro-11β-hydroxy-16α-methyl-17α-[(4-methyl-1,3-thiazole-5-carbonyl)oxy]-3-oxo-androsta-1,4-diene-17β-carbothioic acid *S*-fluoromethyl ester within the cell layer compared to fluticasone propionate.

Examples of disease states in which the formulations of the invention have utility include inflammatory conditions of the nose, throat or lungs such as asthma (including allergen-induced asthmatic reactions), rhinitis (including hayfever), chronic obstructive pulmonary disease, interstitial lung disease, and fibrosis.

It will be appreciated by those skilled in the art that reference herein to treatment extends to prophylaxis as well as the treatment of established conditions.

As mentioned above, compounds of formula (I) are useful in human or veterinary medicine, in particular as anti-inflammatory and anti-allergic agents.

There is thus provided as a further aspect of the invention a formulation according to the first aspect of the invention for use in human or veterinary medicine, particularly in the treatment of patients with inflammatory and/or allergic conditions, especially for treatment once-per-day.

The formulation of the invention may be formulated for administration in any convenient way, and the invention therefore also includes within its scope pharmaceutical compositions comprising a compound of formula (I) or physiologically acceptable salt or solvate thereof together, if desirable, in admixture with one or more physiologically acceptable diluents or carriers.

Further, there is provided a process for the preparation of such pharmaceutical compositions which comprises mixing the ingredients.

Advantageously compositions for topical administration to the lung include dry powder compositions and spray compositions.

Dry powder compositions for topical delivery to the lung may, for example, be presented in pre-metered capsules and cartridges for use in an inhaler or insufflator of, for example, gelatine. Packaging of the formulation may be suitable for unit dose or multi-dose delivery. In the case of multi-dose delivery, the formulation can be pre-metered (eg. as in Diskus, see GB 2242134 or Diskhaler, see GB 2178965, 2129691 and 2169265) or metered in use (eg. as in Turbuhaler, see EP 69715). An example of a unit-dose device is Rotahaler (see GB 2064336). The Diskus inhalation device comprises an elongate strip formed from a base sheet having a plurality of recesses spaced along its length and a lid sheet hermetically but peelably sealed thereto to define a plurality of containers, each container having therein an inhalable formulation containing a formulation according to the first aspect of the invention, preferably combined with lactose. Preferably, the strip is sufficiently flexible to be wound into a roll. The lid sheet and base sheet will preferably have leading end portions which are not sealed to one another and at least one of the said leading end portions is constructed to be attached to a winding means. Also, preferably the hermetic seal between the base and lid sheets extends over their whole width. The lid sheet may preferably be peeled from the base sheet in a longitudinal direction from a first end of the said base sheet.

The dry powder formulation may be presented as blends or or composite particles, or as blends of composite particles. The blended formulations generally contain micronized separate particles of active agent(s) and a suitable powder base such as lactose or starch. Use of lactose is preferred. The powder base acts as a diliuent allowing the actives to be effectively metered, as well as providing and inproving the flow properties of the dry powder composition. Blends may also contain additional components, such as flow enhances, chemical or physical stabilizers, diluents, and flavor masking agents, as will be appreciate by those of ordinary skill in the art. Each capsule or cartridge may generally contain between 20µg-10mg of the compound of formula (I) and a long acting β₂-adrenoreceptor agonist.

Composite particles may include, in any given particle within a powder composition, one or more active agents, and optionally, one or more further excipients. Such excipeints may be suitable for sustaining or controlling the release of the active agents from the powder particle containg such active agent (for example, such as through using of hydrophobic or hydrophylic coating or matrixing agents in the dry powder particles). Additionally, or alternatively, the excipients may increase the physical and/or chemical stability or the medicament particles, and/ or increase powder flow properties or the powder composition, thus making the dry powder more dispersible. Such excipients may form a matrix with the active or active agents, or may coat or be coated by the active or active agents. Additionally, and alternativelty, the composite particles may be formulated with particles having a different composition, thus forming a blend of particles presented as a dry powder composition.

Alternatively, the compound of the invention may be presented without excipients.

Such dry powders may be fromed by micronization, bead milliing, and known particle formulation or co-precipitation techniques (such as single or double emulsion techiques) spray drying, spray coating, sonocrystalization, and the like.

In addition to these dry powder compositions, the compounds of the present invention may be presented as spray composition. Spray compositions may for example be formulated as aqueous solutions or suspensions or as aerosols delivered from pressurised packs, such as a metered dose inhaler, with the use of a suitable liquefied propellant. Aerosol compositions suitable for inhalation can be either a suspension or a solution and generally contain the compound of formula (I) optionally in combination with another therapeutically active ingredient and a suitable propellant such as a fluorocarbon or hydrogen-containing chlorofluorocarbon or mixtures thereof, particularly hydrofluoroalkanes, especially 1,1,1,2-tetrafluoroethane, 1,1,1,2,3,3,3-heptafluoro-n-propane or a mixture thereof. The aerosol composition may optionally contain additional formulation excipients well known in the art such as surfactants e.g. oleic acid or lecithin and cosolvents e.g. ethanol. One example formulation is excipient free and consists essentially of (eg consists of) a compound of formula (I) (optionally together with another active ingredient) and a propellant selected from 1,1,1,2-tetrafluoroethane, 1,1,1,2,3,3,3-heptafluoro-n-propane and mixture thereof. Another example formulation comprises particulate compound of formula (I), a propellant selected from 1,1,1,2-tetrafluoroethane, 1,1,1,2,3,3,3-heptafluoro-n-propane and mixture thereof and a suspending agent which is soluble in the propellant eg an oligolactic acid or derivative thereof as described in WO94/21229. The preferred propellant is 1,1,1,2-tetrafluoroethane. Pressurised formulations will generally be retained in a canister (eg an aluminium canister) closed with a valve (eg a metering valve) and fitted into an actuator provided with a mouthpiece.

Medicaments for administration by inhalation desirably have a controlled particle size. The optimum particle size for inhalation into the bronchial system is usually 1-10µm, preferably 2-5µm. Particles having a size above 20µm are generally too large when inhaled to reach the small airways. To achieve these particle sizes the particles of compound of formula (I) (and any further therapeutically active ingredient) as produced may be size reduced by conventional means eg. by micronisation. The desired fraction may be separated out by air classification or sieving. Preferably, the particles will be crystalline, prepared for example by a process which comprises mixing in a continuous flow cell in the presence of ultrasonic radiation a flowing solution of compound of formula (I) as medicament in a liquid solvent with a flowing liquid antisolvent for said medicament (eg as described in International Patent Application PCT/GB99/04368) or else by a process which comprises admitting a stream of solution of the substance in a liquid solvent and a stream of liquid antisolvent for said substance tangentially into a cylindrical mixing chamber having an axial outlet port such that said streams are thereby intimately mixed through formation of a vortex and precipitation of crystalline particles of the substance is thereby caused (eg as described in International Patent Application PCT/GB00/04327). When an excipient such as lactose is employed, generally, the particle size of the excipient will be much greater than the inhaled medicament within the present invention. When the excipient is lactose it will typically be present as milled lactose, wherein not more than 85% of lactose particles will have a MMD of 60-90µm and not less than 15% will have a MMD of less than 15µm.

Formulations for administration topically to the nose include pressurised aerosol formulations and aqueous formulations administered to the nose by pressurised pump.

Aqueous formulations for administration to the lung or nose may be provided with conventional excipients such as buffering agents, tonicity modifying agents and the like. Aqueous formulations may also be administered to the nose by nebulisation.

If appropriate, the formulations of the invention may be buffered by the addition of suitable buffering agents.

The proportion of the active compound of formula (I) in the formulations according to the invention depends on the precise type of formulation to be prepared but will generally be within the range of from 0.001 to 10% by weight. Generally, however for most types of preparations advantageously the proportion used will be within the range of from 0.005 to 1% and preferably 0.01 to 0.5%. However, in powders for inhalation or insufflation the proportion used will usually be within the range of from 0.1 to 5%.

Aerosol formulations are preferably arranged so that each metered dose or "puff" of aerosol contains 1µg-2000µg eg 20µg-2000µg, preferably about 20µg-500µg of a compound of formula (I) Administration may be once daily or several times daily, for example 2, 3, 4 or 8 times, giving for example 1, 2 or 3 doses each time. Preferably the compound of formula (I) is delivered once or twice daily, especially once per day. The overall daily dose with an aerosol will typically be within the range 10µg-10mg eg 100µg-10mg preferably, 200µg-2000µg.

The formulations are preferably arranged so that each metered dose or puff contains 0.5µg to 1000µg, preferably about 5µg to 500µg of long-acting β₂-adrenoreceptor agonist.

The ratio of the long-acting β₂-adrenoreceptor agonist to the compound of formula (I) is preferably within the range 4:1 to 1:20.

Each metered dose or actuation of the inhaler preferably will contain from 10µg to 100µg of the long-acting β₂-adrenoreceptor agonist and from 25µg to 500µg of a compound of formula (I).

Typically, each metered dose will contain about 25µg of long acting B₂-adrenoreceptor agonist and about 250µg of a compound of formula (I) per 50µl actuation.

Since the compound of formula (I) is long-acting, preferably the composition comprising the compound of formula (I) and the long-acting β₂-adrenoreceptor agonists (M) will be delivered once-per-day and the dose of each will be selected so that the composition has a therapeutic effect in the treatment of respiratory disorders effect (eg in the treatment of asthma or COPD, particularly asthma) over 24 hours or more.

The pharmaceutical formulation according to the invention may also be used in combination with another therapeutically active agent, for example, an anti-histamine or an anti-allergic. Examples of anti-histamines include methapyrilene or loratadine

Other suitable combinations include, for example, other anti-inflammatory agents eg. NSAIDs (eg. PDE4 inhibitors, leukotriene antagonists, iNOS inhibitors, tryptase and elastase inhibitors, beta-2 integrin antagonists and adenosine 2a agonists)) or anti-infective agents (eg. antibiotics, antivirals).

The formulation according to the invention in combination with another therapeutically active ingredient as described above may be formulated for administration in any convenient way, and the invention therefore also includes within its scope pharmaceutical compositions comprising the compound of formula (I) or a physiologically acceptable solvate thereof in combination with another therapeutically active ingredient together, if desirable, in admixture with one or more physiologically acceptable diluents or carriers. The preferred route of administration for inflammatory disorders of the respiratory tract will generally be administration by inhalation.

Further, there is provided a process for the preparation of such pharmaceutical compositions which comprises mixing the ingredients.

The individual compounds of such combinations may be administered either sequentially in separate pharmaceutical compositions as well as simultaneously in combined pharmaceutical formulations. Appropriate doses of known therapeutic agents will be readily appreciated by those skilled in the art.

The compounds of formula (I) and salts and solvates thereof may be prepared by the methodology described hereinafter, constituting a further aspect of this invention.

A process according to the invention for preparing a compound of formula (I) or a salt or solvate thereof comprises alkylation of a thioacid of formula (II) 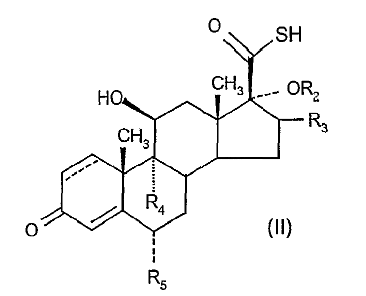 wherein R₂, R₃, R₄, R₅ and 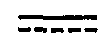 are as defined above,
or a salt thereof.

In this process the compound of formula (II) may be reacted with a compound of formula R₁-L wherein L represents a leaving group such as halogen atom or a tosyl or mesyl group or the like, for example, an appropriate alkyl or haloalkyl halide under standard conditions.

Compounds of formula (II) may conveniently be employed as salts when such salts may be prepared in crystalline form.

When R₁ represents fluoromethyl, the preferred haloalkyl halide reagent is bromofluoromethane.

In a preferred process for preparing a compound of formula (I), a compound of formula (II) or a salt thereof may be treated with bromofluoromethane optionally in the presence of a phase transfer catalyst and optionally in the presence of an added base. A preferred solvent is methylacetate, or more preferably ethylacetate, optionally in the presence of water. The presence of water improves solubility of both starting material and product and the use of a phase transfer catalyst results in an increase in rate of reaction. Examples of phase transfer catalysts that may be employed include (but are not restricted to) tetrabutylammonium bromide, tetrabutylammonium chloride, benzyltributylammonium bromide, benzyltributylammonium chloride, benzyltriethylammonium bromide, methyltributylammonium chloride and methyltrioctylammonium chloride. THF may also advantageously be employed as solvent for the phase transfer catalyst.

Compounds of formula (II) may be prepared from the corresponding 17α-hydroxyl derivative of formula (III): 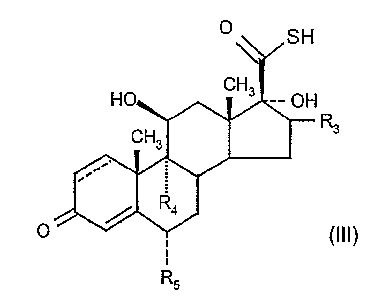 or a salt thereof
wherein R₃, R₄, R₅ and 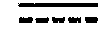 are as defined above, using for example, the methodology described by G. H. Phillipps et al., (1994) Journal of Medicinal Chemistry, 37, 3717-3729. For example the step typically comprises the addition of a reagent suitable for performing the esterification to the ester such as an aryl or heteroarylcarbonyl halide eg. 2-furanoyl chloride in the presence of a mild base eg. triethylamine. Generally the aryl or heteroarylcarbonyl halide would be employed in at least 2 times molar quantity relative to the compound of formula (III). The second mole of aryl or heteroarylcarbonyl halide tends to react with the thioacid moiety in the compound of formula (III) and would need to be removed by reaction with an amine such as diethylamine.

Compounds of formula (III) may be prepared in accordance with procedures described in GB 2088877B.

Compounds of formula (III) wherein R₃ represents methyl in the α configuration, 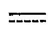 represents a double bond and R₄ and R₅ represent F may also be prepared by a process comprising the following steps:

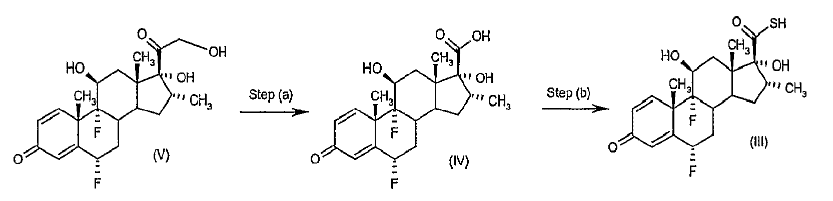

Step (a) comprises oxidation of a solution containing the compound of formula (V). Preferably, step (a) will be performed in the presence of a solvent comprising methanol, water, tetrahydrofuran, dioxan or diethylene glygol dimethylether. For example, so as to enhance yield and throughput, preferred solvents are methanol, water or tetrahydrofuran, and more preferably are water or tetrahydrofuran, especially water and tetrahydrofuran as solvent. Dioxan and diethylene glygol dimethylether are also preferred solvents which may optionally (and preferably) be employed together with water. Preferably, the solvent will be present in an amount of between 3 and 10vol relative to the amount of the starting material (1wt.), more preferably between 4 and 6 vol., especially 5 vol. Preferably the oxidising agent is present in an amount of 1-9 molar equivalents relative to the amount of the starting material. For example, when a 50% w/w aqueous solution of periodic acid is employed, the oxidising agent may be present in an amount of between 1.1 and 10wt. relative to the amount of the starting material (1wt.), more preferably between 1.1 and 3wt., especially 1.3wt. Preferably, the oxidation step will comprise the use of a chemical oxidising agent. More preferably, the oxidising agent will be periodic acid or iodic acid or a salt thereof. Most preferably, the oxidising agent will be periodic acid or sodium periodate, especially periodic acid. Alternatively (or in addition), it will also be appreciated that the oxidation step may comprise any suitable oxidation reaction, eg. one which utilises air and/or oxygen. When the oxidation reaction utilises air and/or oxygen, the solvent used in said reaction will preferably be methanol. Preferably, step (a) will involve incubating the reagents at room temperature or a little warmer, say around 25 °C eg for 2 hours. The compound of formula (I) may be isolated by recrystallisation from the reaction mixture by addition of an anti-solvent. A suitable anti-solvent for compound of formula (I) is water. Surprisingly we have discovered that it is highly desirable to control the conditions under which the compound of formula (IV) is precipitated by addition of anti-solvent eg water. When the recrystallisation is performed using chilled water (eg water/ice mixture at a temperature of 0-5 °C) although better anti-solvent properties may be expected we have found that the crystalline product produced is very voluminous, resembles a soft gel and is very difficult to filter. Without being limited by theory we believe that this low density product contains a large amount of solvated solvent within the crystal lattice. By contrast when conditions of around 10 °C or higher are used (eg around ambient temperature) a granular product of a sand like consistency which is very easily filtered is produced. Under these conditions, crystallisation typically commences after around 1 hour and is typically completed within a few hours (eg 2 hours). Without being limited by theory we believe that this granular product contains little or no of solvated solvent within the crystal lattice.

Step (b) will typically comprise the addition of a reagent suitable for converting a carboxylic acid to a carbothioic acid eg. using hydrogen sulphide gas together with a suitable coupling agent eg. carbonyldiimidazole (CDI) in the presence of a suitable solvent eg. dimethylformamide.

The aforementioned methodology may be adapted for the preparation of other compounds of formula (III).

An alternative process for preparing certain compounds of formula (II) comprises treating a compound of formula (X) with a reagent suitable for converting a carboxylic acid to a carbothioic acid eg using hydrogen sulphide gas together with a suitable coupling agent such as CDI in the presence of a suitable solvent eg DMF. Compounds of formula (X) may be prepared by methodology analogous to that described herein. Other compounds of formula (II) may be prepared similarly.

An alternative process for preparing a compound of formula (I) wherein R₄ represents fluorine or a salt or solvate thereof comprises reacting a compound of formula (VI) 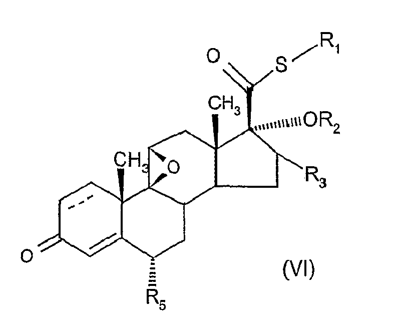 with a fluorine source.

Examples of suitable sources of fluorine include fluoride (eg sodium fluoride) or, more preferably, HF. The preferred reagent is aqueous HF. A solvent such as THF or DMF may be employed.

A compound of formula (VI) may be prepared by a process comprising
(a) alkylating a compound of formula (VII) 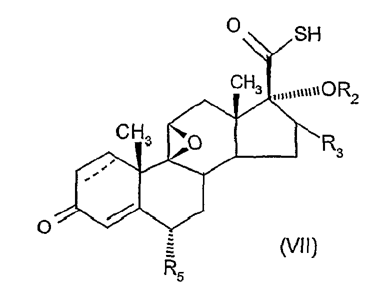 or a salt thereof;
(b) reacting a compound of formula (VIII) 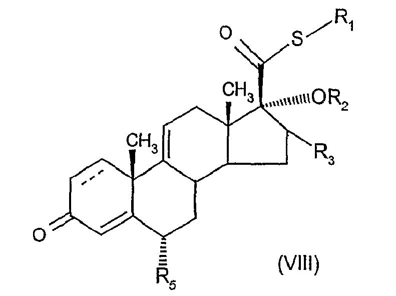 with an epoxide forming reagent; or
(c) esterifying a compound of formula (IX) 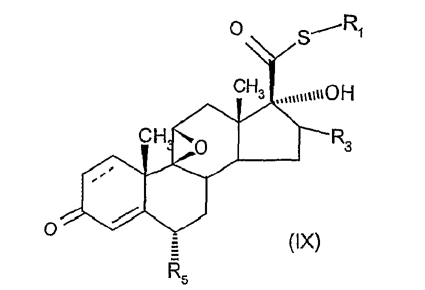 In process (a), analogous conditions to those described above for the conversion of a compound of formula (II) to a compound of formula (I) may be employed. Typically compound of formula (VII) will be reacted with a compound of formula R₁-L wherein L represents a leaving group (eg a halogen atom, a mesyl or tosyl group or the like) for example, an appropriate fluoromethyl halide under standard conditions. Preferably, the fluoromethyl halide reagent is bromofluoromethane.

Process (b) is preferably performed in two steps: (i) formation of a halohydrin especially a bromohydrin (eg by reaction with bromodan or equivalent reagent), followed by (ii) treatment with base such as sodium hydroxide so as to effect ring closure. The product of step (i) is a compound of formula (IXA) which is a novel intermediate that may be isolated, if desired: 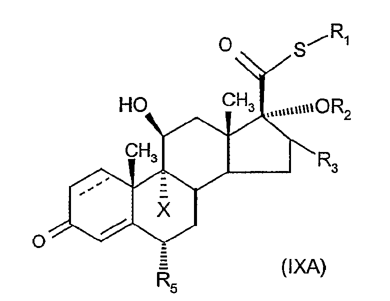 wherein X represents halogen, especially Br.

In process (c), a suitable reagent would be an activated derivative of an aryl or heteroaryl carboxylic acid such as an activated ester or preferably an acid halide eg an acid chloride in the presence of an organic base eg triethylamine. This reaction may be performed at elevated temperature eg around 60 °C or else at ambient temperature in the presence of an acylation catalyst eg dimethylamino pyridine (DMAP).

Compounds of formula (VII) may be prepared by a process comprising esterification of a compound of formula (XI)

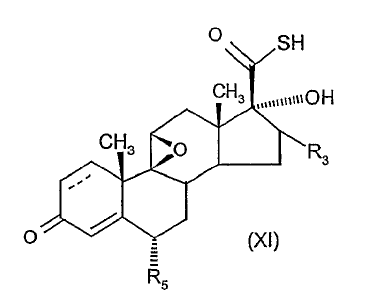

Analogous conditions to those described above for the conversion of a compound of formula (III) to a compound of formula (II) may be employed. For example, a suitable reagent would be an activated derivative of an aryl or heteroaryl carboxylic acid such as an activated ester or preferably an acid halide eg acid chloride in the presence of an organic base eg triethylamine. Certain compounds of formula (XI) are known (J Labelled Compd Radiopharm (1997) 39(7) 567-584) and others may be prepared by analogous methods.

A compound of formula (VIII) may be prepared by a process comprising
(a) alkylating a compound of formula (XII) 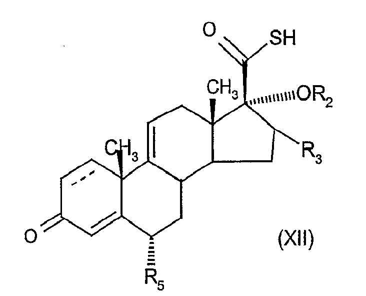 or a salt thereof; or
(b) esterifying a compound of formula (XIII) 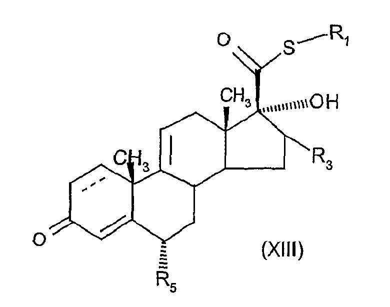 In process (a), analogous conditions to those described above for the conversion of a compound of formula (II) to a compound of formula (I) may be employed. Typically compound of formula (XII) will be reacted with a compound of formula R₁-Lwherein L represents a leaving group (eg a halogen atom, a mesyl or tosyl group or the like) for example, an appropriate fluoromethyl halide under standard conditions. Preferably, the fluoromethyl halide reagent is bromofluoromethane.

In process (b), analogous conditions to those employed above for the conversion of a compound of formula (IX) to a compound of formula (VI) may be employed. For example, a suitable reagent would be an activated derivative of an aryl or heteroaryl carboxylic acid such as an activated ester or preferably an acid halide eg acid chloride in the presence of an organic base eg triethylamine.

Compounds of formula (IX) and (XIII) may be prepared by alkylating the corresponding thioacids (XI) and (XIV) (defined below) using methodology analogous to that already described (eg by reaction with a compound of formula FCH₂L wherein L represents a leaving group (eg a halogen atom, a mesyl or tosyl group or the like) for example, an appropriate fluoromethyl halide under standard conditions. Preferably, the fluoromethyl halide reagent is bromofluoromethane. The thioacids (XI) are either known compounds (J Labelled Compd Radiopharm (1997) 39(7) 567-584) or may be prepared by analogous methods.

Compounds of formula (XII) may be prepared by a process comprising esterifying a compound of formula (XIV): 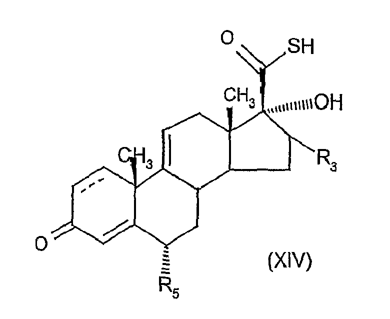 or a salt thereof.
This process may be performed using methodology analogous to that already described. For example, a suitable reagent would be an activated derivative of an aryl or heteroaryl carboxylic acid such as an activated ester or preferably an acid halide eg and acid chloride in the presence of an organic base eg triethylamine.

Compounds of formula (XIV) may be prepared from the corresponding carboxylic acid eg by a process analogous to that described above for the conversion of a compound of formula (IV) to a compound of formula (III). The aforesaid the corresponding carboxylic acid is either known (Upjohn WO90/15816) or may be prepared by conventional methods.

A further alternative process for preparing a compound of formula (I) or a salt or solvate thereof comprises deprotecting or unmasking a compound of formula (I) in which the 11-β-hydroxy group is protected or masked. A first such process comprises deprotecting a compound of formula (XV) 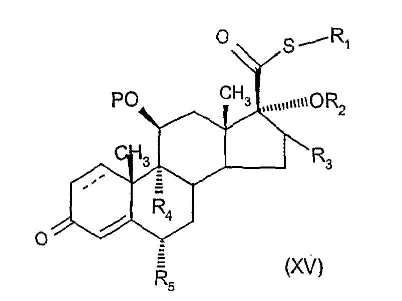 wherein P represents a hydroxy protecting group.

Examples of hydroxy protecting groups P are described in Protective Groups in Organic Chemistry Ed JFW McOmie (Plenum Press 1973) or Protective Groups in Organic Synthesis by Theodora W Green (John Wiley and Sons, 1991).

Examples of suitable hydroxy protecting groups P include groups selected from carbonate, alkyl (eg t-butyl or methoxymethyl), aralkyl (eg benzyl, p-nitrobenzyl, diphenylmethyl or triphenylmethyl), heterocyclic groups such as tetrahydropyranyl, acyl (eg acetyl or benzyl) and silyl groups such as trialkylsilyl (eg t-butyldimethylsilyl). The hydroxy protecting groups may be removed by conventional techniques. Thus, for example, carbonate may be removed by treatment with base and alkyl, silyl, acyl and heterocyclic groups may be removed by solvolysis eg by hydrolysis under acid or basic conditions. Aralkyl groups such as triphenylmethyl may similarly be removed by solvolysis eg by hydrolysis under acidic conditions. Aralkyl groups such as benzyl or p-nitrobenzyl may be cleaved by hydrogenolysis in the presence of a Noble metal catalyst such as palladium on charcoal. p-Nitrobenzyl may also be cleaved by photolysis.

The 11-β-hydroxy group may be masked as a carbonyl group. Thus a second such process comprises reduction of a compound of formula (XVI) 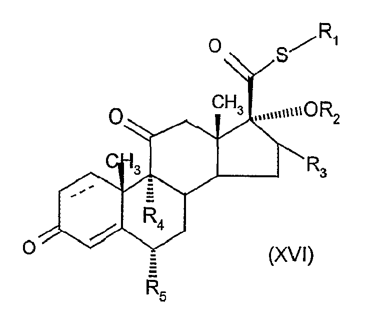 Reduction to the compound of formula (I) may be achieved eg by treatment with a hydride reducing agent such as borohydride eg sodium borohydride

The 11-ketone (XVI) may also be masked. Examples of masked derivatives of compound of formula (XVI) include (i) ketal derivatives eg ketals formed by treatment of the compound of formula (XVI) with an alcohol eg methanol, ethanol or ethan-1,2-diol, (ii) dithioketal derivatives eg dithioketals formed by treatment of a compound of formula (XVI) with a thiol eg methanethiol, ethanethiol or ethan-1,2-dithiol, (iii) monothioketal derivatives eg monothioketals formed by treatment of a compound of formula (XVI) with eg 1-hydroxy-ethane-2-thiol, (iv) derivatives formed by treatment of a compound of formula (XVI) with an alcoholamine eg ephedrine, (v) imines formed by treatment of a compound of formula (XVI) with amines, (vi) oximes formed by treatment of compounds of formula (XVI) with hydroxylamines. We claims such derivatives of compounds of formula (XVI) as an aspect of the invention.

These masked derivatives may be converted back to the ketone by conventional means eg ketals, imines and oximes are converted to carbonyl by treatment with dilute acid and dithioketals are converted to the ketone by a variety of methods as described by P. C. Bulman Page et al (1989), Tetrahedron, 45, 7643-7677 and references therein.

Compounds of formula (XV) may be prepared by a process comprising:
(a) alkylating a compound of formula (XVII) 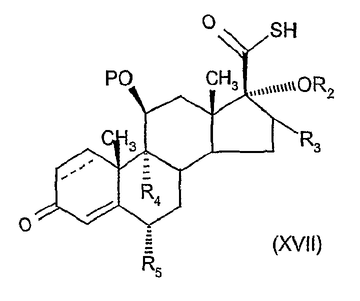 or a salt thereof wherein P represents a hydroxy protecting group; or
(b) esterifying a compound of formula (XVIII) 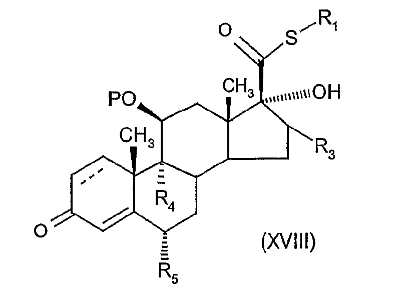 In step (a), analogous conditions to those described above for the conversion of a compound of formula (II) to a compound of formula (I) may be employed. Typically compound of formula (XVII) will be reacted with a compound of formula R₁-Lwherein L represents a leaving group (eg a halogen atom, a mesyl or tosyl group or the like) for example, an appropriate fluoromethyl halide under standard conditions. Preferably, the fluoromethyl halide reagent is bromofluoromethane.

In step (b), analogous conditions to those employed above for the conversion of a compound of formula (IX) to a compound of formula (VI) may be employed. For example, a suitable reagent would be an activated derivative of an aryl or heteroaryl carboxylic acid such as an activated ester or preferably an acid halide eg acid chloride in the presence of an organic base eg triethylamine.

Compound of formula (XVIII) may be prepared by alkylating the corresponding thioacid using methodology analogous to that already described (eg by reaction with a compound of formula R₁-L wherein L represents a leaving group (eg a halogen atom, a mesyl or tosyl group or the like) for example, an appropriate fluoromethyl halide under standard conditions. When R₁ represents -CH₂F, preferably, the fluoromethyl halide reagent is bromofluoromethane. The corresponding thioacids are known compounds or may be prepared by known methods. Compound of formula (XVIII) may alternatively be prepared by protection of the corresponding hydroxy derivative.

Compound of formula (XVII) may be prepared by a process comprising esterifying a compound of formula (XIX) 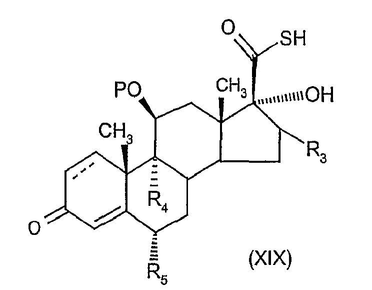 or a salt thereof wherein P represents a hydroxy protecting group.

This process may be performed using methodology analogous to that already described. For example, a suitable reagent would be an activated derivative of an aryl or heteroaryl carboxylic acid such as an activated ester or preferably an acid halide eg acid chloride in the presence of an organic base eg triethylamine.

Compounds of formula (XIX) may be prepared by protecting the corresponding hydroxy derivative, having first protected the thioacid which would need to be subsequently deprotected. The corresponding hydroxy derivatives are known compounds or may be prepared by known methods.

Compounds of formula (XVI) may be prepared by a process comprising:
(a) alkylating a compound of formula (XX) 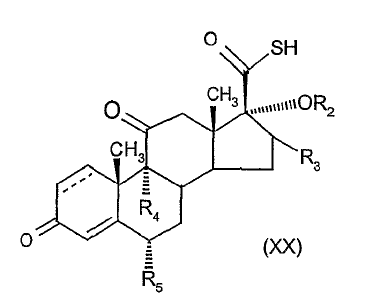 or a salt thereof or a derivative wherein the 11-carbonyl group is masked; or
(b) esterifying a compound of formula (XXI) 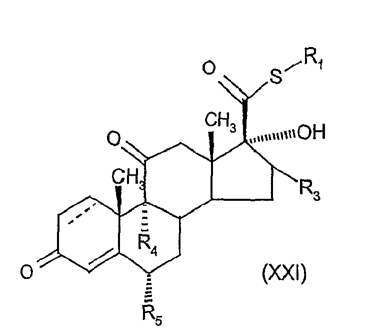 or a derivative wherein the 11-carbonyl group is masked.

In step (a), analogous conditions to those described above for the conversion of a compound of formula (III) to a compound of formula (II) may be employed. Typically compound of formula (XX) will be reacted with a compound of formula R₁-L wherein L represents a leaving group (eg a halogen atom, a mesyl or tosyl group or the like) for example, an appropriate fluoromethyl halide under standard conditions. Preferably, the fluoromethyl halide reagent is bromofluoromethane.

In step (b), analogous conditions to those employed above for the conversion of a compound of formula (IX) to a compound of formula (VI) may be employed. For example, a suitable reagent would be an activated derivative of an aryl or heteroaryl carboxylic acid such as an activated ester or preferably an acid halide eg acid chloride in the presence of an organic base eg triethylamine.

Compound of formula (XXI) or a derivative thereof wherein the 11-ketone group is masked may be prepared by alkylating the corresponding thioacid using methodology analogous to that already described (eg by reaction with a compound of formula R₁-L
wherein L represents a leaving group (eg a halogen atom, a mesyl or tosyl group or the like) for example, an appropriate fluoromethyl halide under standard conditions. Preferably, the fluoromethyl halide reagent is bromofluoromethane. The corresponding thioacids are known compounds.

Compound of formula (XX) may be prepared by a process comprising esterifying a compound of formula (XXII) 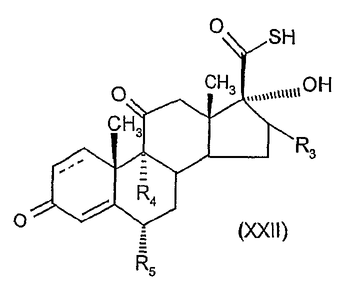 or a salt thereof or a derivative thereof wherein the 11-ketone group is masked.

This process may be performed using methodology analogous to that already described. For example, a suitable reagent would be an activated derivative of an aryl or heteroaryl carboxylic acid such as an activated ester or preferably an acid halide eg acid chloride in the presence of an organic base eg triethylamine.

Compounds of formula (XXII) and derivatives thereof wherein the 11-ketone is masked may be prepared by oxidation of the corresponding hydroxy derivative (IV) (or analogue thereof) followed by masking of the ketone and subsequent conversion of the carboxylic acid group to the thioacid (see eg conversion of compounds of formula (IV) to (III)).

A further alternative process for the preparation of compounds of formula (I) wherein R₁ represents -CH₂F comprises reaction of a compound of formula (XXIII) 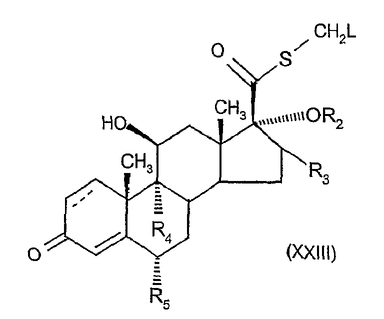 wherein L represents a leaving group (eg halide other than fluoride such as chloride, iodide or a sulphonate ester such mesylate, tosylate, triflate)
with a fluorine source.

Preferably the fluorine source is fluoride ion eg KF. Further details for this conversion may be obtained by reference to G. H. Phillipps et al., (1994) Journal of Medicinal Chemistry, 37, 3717-3729 or J Labelled Compd Radiopharm (1997) 39(7) 567-584).

Some compounds of formula (XXIII) are compounds of formula (I). Compounds of formula (XXIII) may be prepared by methods analogous to those described herein. Corresponding novel intermediates of formula (VI), (VIII), (IX), (IXA), (XV) and (XVI) wherein the -CH₂F moiety is replaced with a -CH₂L moiety (wherein L represents a leaving group other than fluorine) are claimed as an aspect of the invention.

A further alternative process for the preparation of compounds of formula (I) or a solvate thereof comprises deprotection or unmasking of a derivative of a compound of formula (I) in which the 3-carbonyl group is protected or masked.

The 3-carbonyl group may be masked in a manner analogous to that described above in relation to masking of the 11-carbonyl position. Thus the 3-carbonyl may be masked eg as a ketal, monothioketal, dithioketal, derivative with an alcoholamine, oxime or imine. The carbonyl group may be recovered by conventional means eg ketals are converted to carbonyl by treatment with dilute acid and dithioketals are converted to the ketone by a variety of methods as described by P. C. Bulman Page et al (1989), Tetrahedron, 45, 7643-7677 and references therein.

Certain intermediate compounds are new and we provide these, together where appropriate with their salts and solvates, as an aspect of the invention.

The advantages of the compounds of formula (I) and/or its solvates may include the fact that the substance appears to demonstrate excellent anti-inflammatory properties, with predictable pharmacokinetic and pharmacodynamic behaviour, with an attractive side-effect profile, long duration of action, and is compatible with a convenient regime of treatment in human patients, in particular being amenable to once-per day dosing. The advantages may be appreciated in particular when the compound of formula (I) and/or its solvates are employed in combination with a the long-acting β₂-adrenoreceptor agonist. Further advantages may include the fact that the substance has desirable physical and chemical properties which allow for ready manufacture and storage.

### The following non-limiting Examples illustrate the invention:

### EXAMPLES

### General

### ¹H-nmr spectra were recorded at 400 MHz and the chemical shifts are expressed in ppm relative to tetramethylsilane. The following abbreviations are used to describe the multiplicities of the signals: s (singlet), d (doublet), t (triplet), q (quartet), m (multiplet), dd (doublet of doublets), dt (doublet of triplets) and b (broad). Biotage refers to prepacked silica gel cartridges containing KP-Sil run on flash 12i chromatography module. LCMS was conducted on a Supelcosil LCABZ+PLUS column (3.3 cm x 4.6 mm ID) eluting with 0.1% HCO₂H and 0.01 M ammonium acetate in water (solvent A), and 0.05% HCO₂H 5% water in acetonitrile (solvent B), using the following elution gradient 0-0.7 min 0%B, 0.7-4.2 min 100%B, 4.2-5.3 min 0%B, 5.3-5.5 min 0%B at a flow rate of 3 ml/min. The mass spectra were recorded on a Fisons VG Platform spectrometer using electrospray positive and negative mode (ES+ve and ES-ve).

### Intermediates

### Intermediate 1: 6α, 9α-Difluoro-17α-[(2-furanylcarbonyl)oxy]-11β-hydroxy-16α-methyl-3-oxo-androsta-1,4-diene-17β-carbothioic acid

A solution of 6α, 9α-difluoro-11β, 17α-dihydroxy-16α-methyl-3-oxo-androsta-1,4-diene-17β-carbothioic acid (prepared in accordance with the procedure described in GB 2088877B) (18g, 43.64mmol) in anhydrous dichloromethane (200ml) and triethylamine (15.94ml, 114mmol) was treated at <5 °C with a solution of 2-furoyl chloride (11.24ml, 114mmol) in anhydrous dichloromethane (100ml) over approximately 40min. The solution was stirred at <5°C for 30min. The resulting solid was collected by filtration, washed successively with 3.5% aqueous sodium hydrogen carbonate solution, water, 1M hydrochloric acid, and water and dried in vacuo at 60 °C to give a cream coloured solid. The dichloromethane filtrate was washed successively with 3.5% sodium hydrogen carbonate solution, water, 1M hydrochloric acid, water, dried (Na₂SO₄) and evaporated to give a cream coloured solid which was combined with that isolated above. The combined solids (26.9g) were suspended in acetone (450ml) and stirred. Diethylamine (16.8ml, 162mmol) was added and the mixture stirred at room temperature for 4.5h. The mixture was concentrated and the precipitate collected by filtration and washed with a little acetone. The washings and filtrate were combined, concentrated and loaded onto a silica gel Biotage column which was eluted with 24:1 chloroform: methanol. Fractions which contained the more polar component were combined and evaporated to give a cream coloured solid. This was combined with the solid isolated above and dried in vacuo to give a pale beige coloured solid (19.7g). This was dissolved in warm water, the pH adjusted to 2 with concentrated hydrochloric acid and the mixture extracted with ethyl acetate. The organic extract was dried (Na₂SO₄) and evaporated to give, after drying at 50°C, the title compound as a cream coloured solid (18.081 g, 82%): LCMS retention time 3.88min, *m*/*z* 507 MH⁺, NMR δ (CDCl₃) includes 7.61 (1H, m), 7.18 - 7.12 (2H, m), 6.52 (1 H, dd, J 4, 2Hz), 6.46 (1 H, s), 6.41 (1 H, dd, J 10, 2Hz), 5.47 and 5.35 (1 H, 2m), 4.47 (1H, bd, *J* 9Hz), 3.37 (1H, m), 1.55 (3H, s), 1.21 (3H, s), 1.06 (3H, d, *J* 7Hz).

The following intermediates were prepared using a method analogous to that described for Intermediate 1:
Intermediate 2: 6α,9α-Difluoro-17α-[(3-furanylcarbonyl)oxy]-11β-hydroxy-16α-methyl-3-oxo-androsta-1,4-diene-17β-carbothioic acid
   LCMS retention time 3.75min, m/z 507 MH⁺.
Intermediate 3: 6α,9α-Difluoro-11β-hydroxy-16α-methyl-3-oxo-17α-[(2-thienylcarbonyl)oxy]-androsta-1,4-diene-17β-carbothioic acid
   LCMS retention time 3.93min, *m*/*z* 523 MH⁺.
Intermediate 4: 6α,9α-Difluoro-11β-hydroxy-16α-methyl-3-oxo-17α-[(3-thienylcarbonyl)oxy]-androsta-1,4-diene-17β-carbothioic acid
   LCMS retention time 3.95min, m/z 523 MH⁺.
Intermediate 5: 17α-(Benzoyl)oxy-6α,9α-difluoro-11β-hydroxy-16α-methyl-3-oxo-androsta-1,4-diene-17β-carbothioic acid
   LCMS retention time 4.02min, *m*/*z* 517 MH⁺.
Intermediate 6: 9α-Fluoro-17α-[(2-furanylcarbonyl)oxy]-11β-hydroxy-16β-methyl-3-oxo-androsta-1,4-diene-17β-carbothioic acid
Intermediate 6 was prepared from 11β,17α-dihydroxy-9α-fluoro-16β-methyl-3-oxo-androsta-1,4-diene-17β-carbothioic acid (prepared in accordance with the procedure described in Phillips et al, (1994) J. Med. Chem. 37, 3717-3729). LCMS retention time 3.61 min, *m*/*z* 489 MH⁺.
Intermediate 7: 6α,9α-Difluoro-11β-hydroxy-16α-methyl-17α-[(5-methylthiophene-2-carbonyl)oxy]-3-oxo-androsta-1,4-diene-17β-carbothioic acid
   LCMS retention time 4.01 min, *m*/*z* 537 MH⁺
Intermediate 8: 6α,9α-Difluoro-11β-hydroxy-17α-[(isoxazole-5-carbonyl)oxy]-16α-methyl-3-oxo-androsta-1,4-diene-17β-carbothioic acid
   LCMS retention time 3.69min, *m*/*z* 508 MH⁺
Intermediate 9: 17α-[(5-Chlorothiophene-2-carbonyl)oxy]-6α,9α-difluoro-11β-hydroxy-16α-methyl-3-oxo-androsta-1,4-diene-17β-carbothioic acid
   LCMS retention time 4.36min, *m*/*z* 557/559 MH⁺
Intermediate 10: 6α,9α-Difluoro-17α(3,5-dimethylisoxazole-4-carbonyl)oxy]-11β-hydroxy-16α-methyl-3-oxo-androsta-1,4-diene-17β-carbothioic acid
   LCMS retention time 3.71 min, *m*/*z* 536 MH⁺
Intermediate 11: 17α-[(5-Chloro-4-methoxy-thiophene-3-carbonyl)oxyl-6α,9α-difluoro-11β-hydroxy-16α-methyl-3-oxo-androsta-1,4-diene-17β-carbothioic acid
   LCMS retention time 4.18min, *m*/*z* 587/589 MH⁺
Intermediate 12: 6α,9α-Difluoro-11β-hydroxy-16α-methyl-17α-[(4-methyl-1,2,3-thiadiazole-5-carbonyl)oxy]-3-oxo-androsta-1,4-diene-17β-carbothioic acid
   LCMS retention time 4.06min, m/z 539 MH⁺
Intermediate 13: 17α-[(3-Bromothiophene-2-carbonyl)oxy]-6α,9α-difluoro-11β-hydroxy-16α-methyl-3-oxo-androsta-1,4-diene-17β-carbothioic acid
   LCMS retention time 4.31min, *m*/*z* 601/603 MH⁺
Intermediate 14: 17α-[(2,5-Dichlorothiophene-3-carbonyl)oxy]-6α,9α-difluoro-11β-hydroxy-16α-methyl-3-oxo-androsta-1,4-diene-17β-carbothioic acid
   LCMS retention time 4.59min, m/z 591/593/595 MH⁺
Intermediate 15: 17α-[(5-Bromofuran-2-carbonyl)oxy]-6α,9α-difluoro-11β-hydroxy-16α-methyl-3-oxo-androsta-1,4-diene-17β-carbothioic acid
   LCMS retention time 4.14min, *m*/*z* 585/587 MH⁺
Intermediate 16: 6α,9α-Difluoro-17α-[(2,5-dimethylfuran-3-carbonyl)oxy]-11β-hydroxy-16α-methyl-3-oxo-androsta-1,4-diene-17β-carbothioic acid
   LCMS retention time 4.02min, *m*/*z* 535 MH⁺
Intermediate 17: 17α-[(3-Chlorothiophene-2-carbonyl)oxy]-6α,9α-difluoro-11β-hydroxy-16α-methyl-3-oxo-androsta-1,4-diene-17β-carbothioic acid
   LCMS retention time 4.27min, *m*/*z* 557/559 MH⁺
Intermediate 18: 6α,9α-Difluoro-11β-hydroxy-16α-methyl-17α-[(2-methylfuran-3-carbonyl)oxy]-3-oxo-androsta-1,4-diene-17β-carbothioic acid
   LCMS retention time 3.92min, *m*/*z* 521 MH⁺
Intermediate 19: 6α,9α-Difluoro-11β-hydroy-16α-methyl-17α-[(3-methylfuran-2-carbonyl)oxy]-3-oxo-androsta-1,4-diene-17β-carbothioic acid
   LCMS retention time 3.85min, *m*/*z* 521 MH⁺
Intermediate 20: 6α,9α-Difluoro-11β-hydroxy-16α-methyl-17α-[(5-methylisoxazole-4-carbonyl)oxy]-3-oxo-androsta-1,4-diene-17β-carbothioic acid
   LCMS retention time 3.74min, *m*/*z* 522 MH⁺
Intermediate 21: 6α,9α-Difluoro-11β-hydroxy-16α-methyl-17α-[(1-methyl-1H-pyrrole-2-carbonyl)oxy]-3-oxo-androsta-1,4-diene-17β-carbothioic acid
   LCMS retention time 3.78min, *m*/*z* 520 MH⁺
Intermediate 22: 6α,9α-Difluoro-11β-hydroxy-16α-methyl-3-oxo-17α-[(1,3-thiazole-4-carbonyl)oxy]-androsta-1,4-diene-17β-carbothioic acid
   LCMS retention time 3.48min, *m*/*z* 524 MH⁺
Intermediate 23: 6α,9α-Difluoro-17α-[(2,4-dimethyl-1,3-thiazole-5-carbonyl)oxy]-11β-hydroxy-16α-methyl-3-oxo-androsta-1,4-diene-17β-carbothioic acid
   LCMS retention time 3.72min, *m*/*z* 552 MH⁺
Intermediate 24 6α,9α-Difluoro-11β-hydroxy-16α-methyl-17α-[(5-methylisoxazole-3-carbonyl)oxy]-3-oxo-androsta-1,4-diene-17β-carbothioic acid
   LCMS retention time 3.72min, *m*/*z* 522 MH⁺
Intermediate 25: 6α,9α-Difluoro-11β-hydroxy-16α-methyl-17α-[(3-methylisoxazole-5-carbonyl)oxy]-3-oxo-androsta-1,4-diene-17β-carbothioic acid
   LCMS retention time 3.84min, *m*/*z* 522 MH⁺
Intermediate 26: 6α,9α-Difluoro-17α-[(1,3-dimethyl-1H-pyrazole-5-carbonyl)oxy]-11β-hydroxy-16α-methyl-3-oxo-androsta-1,4-diene-17β-carbothioic acid
   LCMS retention time 3.72min, *m*/*z* 535 MH⁺
Intermediate 27: 6α,9α-Difluoro-11β-hydroxy-16α-methyl-3-oxo-17α-[(1,2,3-thiadiazole-5-carbonyl)oxy]-androsta-1,4-diene-17β-carbothioic acid
   LCMS retention time 3.89min, *m*/*z* 525 MH⁺
Intermediate 28: 6α,9α-Difluoro-11β-hydroxy-17α-[(isoxazole-3-carbonyl)oxy]-16α-methyl-3-oxo-androsta-1,4-diene-17β-carbothioic acid
   LCMS retention time 3.78min, *m*/*z* 508 MH⁺
Intermediate 29: 6α,9α-Difluoro-11β-hydroxy-17α-[(4-methoxy-thiophene-3-carbonyl)oxy]-16α-methyl-3-oxo-androsta-1,4-diene-17β-carbothioic acid
   LCMS retention time 3.74min, *m*/*z* 553 MH⁺
Intermediate 30 6α,9α-Difluoro-11β-hydroxy-16α-methyl-17α-[(2-methyl-1,3-thiazole-4-carbonyl)oxy]-3-oxo-androsta-1,4-diene-17β-carbothioic acid
   LCMS retention time 3.70min, *m*/*z* 538 MH⁺
Intermediate 31: 6α,9α-Difluoro-17α-[(3-ethoxy-thiophene-2-carbonyl)oxy]-11β-hydroxy-16α-methyl-3-oxo-androsta-1,4-diene-17β-carbothioic acid
   LCMS retention time 3.85min, *m*/*z* 567 MH⁺
Intermediate 32: 6α,9α-Difluoro-11β-hydroxy-16α-methyl-3-oxo-17α-[(1,2,3-thiadiazole-4-carbonyl)oxy]-androsta-1,4-diene-17β-carbothioic acid
   LCMS retention time 3.70min, *m*/*z* 526 MH⁺
Intermediate 33: 6α,9α-Difluoro-11β-hydroxy-16α-methyl-3-oxo-17α-[(1H-pyrrole-2-carbonyl)oxy]-androsta-1,4-diene-17β-carbothioic acid
   LCMS retention time 3.75min, *m*/*z* 506 MH⁺
Intermediate 34: 6α,9α-Difluoro-11β-hydroxy-16α-methyl-3-oxo-17α-[(1,3-thiazole-5-carbonyl)oxy]-androsta-1,4-diene-17β-carbothioic acid
   LCMS retention time 4.29min, *m*/*z* 524 MH⁺
Intermediate 35: 6α,9α-Difluoro-11β-hydroxy-16α-methyl-3-oxo-17α-[(1,2,5-thiadiazole-3-carbonyl)oxy]-androsta-1,4-diene-17β-carbothioic acid
   LCMS retention time 4.65min, *m*/*z* 525 MH⁺
Intermediate 36: 6α,9α-Difluoro-11β-hydroxy-17α-[(isothiazole-3-carbonyl)oxy]-16α-methyl-3-oxo-androsta-1,4-diene-17β-carbothioic acid
   LCMS retention time 4.44min, m/z 524 MH⁺
Intermediate 37: 6α,9α-Difluoro-11β-hydroxy-17α-[(isothiazole-5-carbonyl)oxy)-16α-methyl-3-oxo-androsta-1,4-diene-17β-carbothioic acid
   LCMS retention time 4.60min, *m*/*z* 524 MH⁺
Intermediate 38: 6α,9α-Difluoro-11β-hydroxy-16α-methyl-17α-[(3-methylthiophene-2 carbonyl)oxy]-3-oxo-androsta-1,4-diene-17β-carbothioic acid
   LCMS retention time 4.31 min, *m*/*z* 537 MH⁺
Intermediate 39: 6α,9α-Difluoro-11β-hydroxy-16α-methyl-17α-[(4-methyl-1,3-thiazole-5-carbonyl)oxy]-3-oxo-androsta-1,4-diene-17β-carbothioic acid
   LCMS retention time 4.19min, *m*/*z* 538 MH⁺
Intermediate 40: 17α-[(1-Ethyl-3-methyl-1H-pyrazole-5-carbonyl)oxy]-6α,9α-difluoro-11β-hydroxy-16α-methyl-3-oxo-androsta-1,4-diene-17β-carbothioic acid
   LCMS retention time 4.33min, *m*/*z* 549 MH⁺
Intermediate 41: 6α,9α-Difluoro-17α-[(1-methyl-1H-imidazole-5-carbonyl)oxy]-11β-hydroxy-16α-methyl-3-oxo-androsta-1,4-diene-17β-carbothioic acid
   LCMS retention time 3.91 min, *m*/*z* 521 MH⁺

### Examples

### Example 1: 6α,9α-Difluoro-17α-[(2-furanylcarbonyl)oxy]-11β-hydroxy-16α-methyl-3-oxo-androsta-1,4-diene-17β-carbothioic acid S-fluoromethyl ester

A suspension of Intermediate 1 (2.5g, 4.94mmol) was dissolved in anhydrous N, N-dimethylformamide (25ml) and sodium hydrogen carbonate (465mg, 5.53mmol) was added. The mixture was stirred at -20°C and bromofluoromethane (0.77ml, 6.37mmol) was added and the mixture was stirred at -20°C for 2h. Diethylamine (2.57ml, 24.7mmole) was added and the mixture stirred at -20°C for 30min. The mixture was added to 2M hydrochloric acid (93ml) and stirred for 30min. Water (300ml) was added and the precipitate was collected by filtration, washed with water and dried in vacuo at 50°C to give a white solid which was recrystallised from acetone/water and dried in vacuo at 50°C to give the title compound (2.351 g, 88%): LCMS retention time 3.66min, m/z 539 MH⁺, NMR δ (CDCl₃) includes 7.60 (1H, m), 7.18 - 7.11 (2H, m), 6.52 (1 H, dd, *J* 4.2Hz), 6.46 (1 H, s), 6.41 (1 H, dd,*J* 10, 2Hz), 5.95 and 5.82 (2H dd, J 51, 9Hz), 5.48 and 5.35 (1 H, 2m), 4.48 (1 H, m), 3.48 (1 H, m), 1.55 (3H, s), 1.16 (3H, s), 1.06 (3H, d, J 7Hz).

### Example 2: 6α,9α-Difluoro-17α-[(3-furanyicarbonyl)oxy]-11β-hydroxy-16α-methyl-3-oxo-androsta-1,4-diene-17β-carbothioic acid S-fluoromethyl ester

Example 2 was prepared from Intermediate 2 using a method analogous to that described for Example 1.
LCMS retention time 3.72min, m/z 539 MH⁺.

### Example 3: 6α,9α-Difluoro-11β-hydroxy-16α-methyl-3-oxo-17α-[(2-thienylcarbonyl)oxy]-androsta-1,4-diene-17β-carbothioic acid S-fluoromethyl ester

Example 3 was prepared from Intermediate 3 using a method analogous to that described for Example 1.
LCMS retention time 3.81 min, m/z 555 MH⁺.

### Example 4: 6α,9α-Difluoro-11β-hydroxy-16α-methyl-3-oxo-17α-[(3-thienylcarbonyl)oxy]-androsta-1,4-diene-17β-carbothioic acid S-fluoromethyl ester

Example 4 was prepared from Intermediate 4 using a method analogous to that described for Example 1.
LCMS retention time 3.82min, *m*/*z* 555 MH⁺.

### Example 5: 17α-(Benzoyl)oxy-6α,9α-Difluoro-11β-hydroxy-16α-methyl-3-oxo-androsta-1,4-diene-17β-carbothioic acid S-fluoromethyl ester

Example 5 was prepared from Intermediate 5 using a method analogous to that described for Example 1.
LCMS retention time 3.73min, *m*/*z* 549 MH⁺.

### Example 6: 9α-Fluoro-17α-[(2-furanylcarbonyl)oxy]-11β-hydroxy-16β-methyl-3-oxo-androsta-1,4-diene-17β-carbothioic acid S-fluoromethyl ester

Example 6 was prepared from Intermediate 6 using a method analogous to that described for Example 1.
LCMS retention time 3.61 min, m/z 521 MH⁺.

### Example 7: 6α,9α-Difluoro-17α-[(2-furanylcarbonyl)oxy]-11β-hydroxy-16α-methyl-3-oxo-androst- 4-ene-17β-carbothioic acid S-fluoromethyl ester

A solution of Example 1 (500mg, 0.93mmol) and Wilkinson's catalyst (150mg) in a mixture of toluene (14ml) and ethanol (7ml) was stirred in an atmosphere of hydrogen for 23h. The solution was evaporated to dryness and the residue purified by Biotage chromatography using ethyl acetate:petroleum ether (1:2) as eluant to give a yellow solid (435mg) which was recrystallised (ethyl acetate/petroleum ether) to give the title compound (364mg, 72%).
LCMS retention time 3.57min, *m*/*z* 541 MH⁺.

### Example 8: 6α,9α-Difluoro-11β-hydroxy-17α-[(isoxazole-5-carbonyl)oxy]-16α-methyl-3-oxo-androsta-1,4-diene-17β-carbothioic acid S-fluoromethyl ester

Example 8 was prepared from Intermediate 8 using a method analogous to that described for Example 1: LCMS retention time 3.47min, *m*/*z* 540 MH⁺

### Example 9: 17α-[(5-Chlorothiophene-2-carbonyl)oxy]-6α,9α-dlfluoro-1β-hydroxy-16α-methyl-3-oxo-androsta-1,4-diene-17β-carbothioic acid S-fluoromethyl ester

Example 9 was prepared from Intermediate 9 using a method analogous to that described for Example 1: LCMS retention time 3.89min, *m*/*z* 589/591 MH⁺

### Example 10: 6α,9α-Difluoro-17α-[(3,5-dlmethylisoxazole-4-carbonyl)oxy]-11β-hydroxy-16α-methyl-3-oxo-androsta-1,4-diene-17β-carbothioic acid S-fluoromethyl ester

Example 10 was prepared from Intermediate 10 using a method analogous to that described for Example 1: LCMS retention time 3.70min, *m*/*z* 568 MH⁺

### Example 11: 17α-[(5-Chloro-4-methoxy-thiophene-3-carbonyl)oxy]-6α,9α-difluoro-11 β-hydroxy-16α-methyl-3-oxo-androsta-1,4-diene-17β-carbothioic acid S-fluoromethyl ester

Example 11 was prepared from Intermediate 11 using a method analogous to that described for Example 1: LCMS retention time 3.99min, *m*/*z* 619/621 MH⁺

### Example 12: 6α,9α-Difluoro-11β-hydroxy-16α-methyl-17α-[(4-methyl-1,2,3-thiadiazole-5-carbonyl)oxy]-3-oxo-androsta-1,4-diene-17β-carbothioic acid S-fluoromethyl ester

Example 12 was prepared from Intermediate 12 using a method analogous to that described for Example 1: LCMS retention time 3.74min, *m*/*z* 571 MH⁺

### Example 13: 17α-[(3-Bromothiophene-2-carbonyl)oxy]-6α,9α-difluoro-11β-hydroxy-16α-methyl-3-oxo-androsta-1,4-diene-17β-carbothioic acid S-fluoromethyl ester

Example 13 was prepared from Intermediate 13 using a method analogous to that described for Example 1: LCMS retention time 3.92min, *m*/*z* 633/635 MH⁺

### Example 14: 17α-[(2,5-Dichlorothiophene-3-carbonyl)oxy]-6α,9α-difluoro-11β-hydroxy-16α-methyl-3-oxo-androsta-1,4-diene-17β-carbothioic acid S-fluoromethyl ester

Example 14 was prepared from Intermediate 14 using a method analogous to that described for Example 1: LCMS retention time 4.17min, *m*/*z* 623/625/627 MH⁺

### Example 15: 17α-[(5-Bromofuran-2-carbonyl)oxy]-6α,9α-difluoro-11β-hydroxy-16α-methyl-3-oxo-androsta-1,4-diene-17β-carbothioic acid S-fluoromethyl ester

Example 15 was prepared from Intermediate 15 using a method analogous to that described for Example 1: LCMS retention time 3.78min, *m*/*z* 615/617 MH⁺

### Example 16: 6α,9α-Difluoro-17α-[(2,5-dimethylfuran-3-carbonyl)oxy]-11β-hydroxy-16α-methyl-3-oxo-androsta-1,4-diene-17β-carbothioic acid S-fluoromethyl ester

Example 16 was prepared from Intermediate 16 using a method analogous to that described for Example 1: LCMS retention time 3.85min, *m*/*z* 576 MH⁺

### Example 17: 17α-[(3-Chlorothiophene-2-carbonyl)oxy]-6α,9α-difluoro-11β-hydroxy-16α-methyl-3-oxo-androsta-1,4-diene-17β-carbothioic acid S-fluoromethyl ester

Example 17 was prepared from Intermediate 17 using method analogous to that described for Example 1: LCMS retention time 3.88min, *m*/*z* 589/591 MH⁺

### Example 18: 6α,9α-Difluoro-11β-hydroxy-16α-methyl-17α-[(2-methylfuran-3-carbonyl)oxy]-3-oxo-androsta-1,4-diene-17β-carbothioic acid S-fluoromethyl ester

Example 18 was prepared from Intermediate 18 using a method analogous to that described for Example 1: LCMS retention time 3.74min, *m*/*z* 553 MH⁺

### Example 19: 6α,9α-Difluoro-11β-hydroxy-16α-methyl-17α-[(3-methylfuran-2-carbonyl)oxy]-3-oxo-androsta-1,4-diene-17β-carbothioic acid S-fluoromethyl ester

Example 19 was prepared from Intermediate 19 using a method analogous to that described for Example 1: LCMS retention time 3.66min, *m*/*z* 553 MH⁺

### Example 20: 6α,9α-Difluoro-11β-hydroxy-16α-methyl-17α-[(5-methylisoxazole-4-carbonyl)oxy]-3-oxo-androsta-1,4-diene-17β-carbothioic acid S-fluoromethyl ester

Example 20 was prepared from Intermediate 20 using a method analogous to that described for Example 1: LCMS retention time 3.60min, *m*/*z* 554 MH⁺

### Example 21: 6α,9α-Difluoro-11β-hydroxy-16α-methyl-17α-[(1-methyl-1H-pyrrole-2-carbonyl)oxy]-3-oxo-androsta-1,4-diene-17β-carbothioic acid S-fluoromethyl ester

Example 21 was prepared from Intermediate 21 using a method analogous to that described for Example 1: LCMS retention time 3.72min, *m*/*z* 552 MH⁺

### Example 22: 6α,9α-Difluoro-11β-hydroxy-16α-methyl-3-oxo-17α-[(1,3-thiazole-4-carbonyl)oxy]-androsta-1,4-diene-17β-carbothioic acid S-fluoromethyl ester

Example 22 was prepared from Intermediate 22 using a method analogous to that described for Example 1: LCMS retention time 3.47min, *m*/*z* 552 MH⁺

### Example 23: 6α,9α-Difluoro-17α-[(2,4-dimethyl-1,3-thiazole-5-carbonyl)oxy]-11β-hydroxy-16α-methyl-3-oxo-androsta-1,4-diene-17β-carbothiolc acid S-fluoromethyl ester

Example 23 was prepared from Intermediate 23 using a method analogous to that described for Example 1: LCMS retention time 3.51 min, *m*/*z* 584 MH⁺

### Example 24: 6α,9α-Difluoro-11β-hydroxy-16α-methyl-17α-[(5-methylisoxazole-3-carbonyl)oxy]-3-oxo-androsta-1,4-diene-17β-carbothioic acid S-fluoromethyl ester

Example 24 was prepared from Intermediate 24 using a method analogous to that described for Example 1: LCMS retention time 3.65min, *m*/*z* 554 MH⁺

### Example 25: 6α,9α-Difluoro-11β-hydroxy-16α-methyl-17α-[(3-methylisoxazole-5-carbonyl)oxy]-3-oxo-androsta-1,4-diene-17β-carbothioic acid S-fluoromethyl ester

Example 25 was prepared from Intermediate 25 using a method analogous to that described for Example 1: ^{L}CMS retention time 3.52min, *m*/*z* 554 MH⁺

### Example 26: 6α,9α-Difluoro-17α-[(1,3-dimethyl-1H-pyrazole-5-carbonyl)oxy]-11β-hydroxy-16α-methyl-3-oxo-androsta-1,4-diene-17β-carbothioic acid S-fluoromethyl ester

Example 26 was prepared from Intermediate 26 using a method analogous to that described for Example 1: LCMS retention time 3.52min, *m*/*z* 567 MH⁺

### Example 27: 6α,9α-Difluoro-11β-hydroxy-16α-methyl-3-oxo-17α-[(1,2,3-thiadiazole-5-carbonyl)oxy]-androsta-1,4-diene-17β-carbothioic acid S-fluoromethyl ester

Example 27 was prepared from Intermediate 27 using a method analogous to that described for Example 1: LCMS retention time 3.47min, *m*/*z* 557 MH⁺

### Example 28: 6α,9α-Difluoro-11β-hydroxy-17α-[(isoxazole-3-carbonyl)oxy]-16α-methyl-3-oxo-androsta-1,4-diene-17β-carbothioic acid S-fluoromethyl ester

Example 28 was prepared from Intermediate 28 using a method analogous to that described for Example 1: LCMS retention time 3.48min, *m*/*z* 540 MH⁺

### Example 29: 6α,9α-Difluoro-11β-hydroxy-17α-[(4-methoxy-thiophene-3-carbonyl)oxy]-16α-methyl-3-oxo-androsta-1,4-diene-17β-carbothioic acid S-fluoromethyl ester

Example 29 was prepared from Intermediate 29 using a method analogous to that described for Example 1: LCMS retention time 3.69min, *m*/*z* 585 MH⁺

### Example 30 6α,9α-Difluoro-11β-hydroxy-16α-methyl-17α-[(2-methyl-1,3-thiazole-4-carbonyl)oxy]-3-oxo-androsta-1,4-diene-17β-carbothioic acid S-fluoromethyl ester

Example 30 was prepared from Intermediate 30 using a method analogous to that described for Example 1: LCMS retention time 3.47min, *m*/*z* 570 MH⁺

### Example 31: 6α,9α-Difiuoro-17α-[(3-ethoxy-thiophene-2-carbonyl)oxy]-11β-hydroxy-16α-methyl-3-oxo-androsta-1,4-diene-17β-carbothioic acid S-fluoromethyl ester

Example 31 was prepared from Intermediate 31 using a method analogous to that described for Example 1: LCMS retention time 3.68min, *m*/*z* 599 MH⁺

### Example 32: 6α,9α-Difluoro-11β-hydroxy-16α-methyl-3-oxo-17α-[(1,2,3-thiadiazole-4-carbonyl)oxy]-androsta-1,4-diene-17β-carbothioic acid S-fluoromethyl ester

Example 32 was prepared from Intermediate 32 using a method analogous to that described for Example 1: LCMS retention time 3.30min, *m*/*z* 557 MH⁺

### Example 33: 6α,9α-Difluoro-11β-hydroxy-16α-methyl-3-oxo-17α-[(1H-pyrrole-2-carbonyl)oxy]-androsta-1,4-diene-17β-carbothioic acid S-fluoromethyl ester

Example 33 was prepared from Intermediate 33 using a method analogous to that described for Example 1: LCMS retention time 3.42min, *m*/*z* 528 MH⁺

### Example 34: 6α,9α-Difluoro-11β-hydroxy-16α-methyl-3-oxo-17α-[(1,3-thiazole-5-carbonyl)oxy]-androsta-1,4-diene-17β-carbothioic acid S-fluoromethyl ester

Example 34 was prepared from Intermediate 34 using a method analogous to that described for Example 1: LCMS retention time 3.44min, *m*/*z* 556 MH⁺

### Example 35: 6α,9α-Difluoro-11β-hydroxy-16α-methyl-3-oxo-17α-[(1,2,5-thiadiazole-3-carbonyl)oxy]-androsta-1,4-diene-17β-carbothioic acid S-fluoromethyl ester

Example 35 was prepared from Intermediate 35 using a method analogous to that described for Example 1: LCMS retention time 3.53min, *m*/*z* 557 MH⁺

### Example 36: 6α,9α-Difluoro-11β-hydroxy-17α-[(isothiazole-3-carbonyl)oxy]-16α-methyl-3-oxo-androsta-1,4-diene-17β-carbothioic acid S-fluoromethyl ester

Example 36 was prepared from Intermediate 36 using a method analogous to that described for Example 1: LCMS retention time 3.51 min, *m*/*z* 556 MH⁺

### Example 37: 6α,9α-Difluoro-11β-hydroxy-17α-[(isothiazole-5-carbonyl)oxy]-16α-methyl-3-oxo-androsta-1,4-diene-17β-carbothioic acid S-fluoromethyl ester

Example 37 was prepared from Intermediate 37 using a method analogous to that described for Example 1: LCMS retention time 3.59min, *m*/*z* 556 MH⁺

### Example 38: 6α,9α-Difluoro-11β-hydroxy-16α-methyl-17α-[(5-methylthiophene-2-carbonyl)oxy]-3-oxo-androsta-1,4-diene-17β-carbothioic acid S-fluoromethyl ester

Example 38 was prepared from Intermediate 7 using a method analogous to that described for Example 1: LCMS retention time 3.78min, *m*/*z* 569 MH⁺

### Example 39: 6α,9α-Difluoro-17α-[(2-furanylcarbonyl)oxy]-11β-hydroxy-16α-methyl-3-oxo-androsta-1,4-diene-17β-carbothioic acid S-methyl ester

A suspension of Intermediate 1 (507mg, 1mmol) was dissolved in anhydrous N, N-dimethylformamide (5ml) and sodium hydrogen carbonate (92mg, 1.1mmol) was added. The mixture was stirred at 0°C and iodomethane (0.125ml, 2mmol) was added and the mixture was stirred at 0-5°C for 2.5h. Diethylamine (0.41ml, 4mmole) was added and the mixture stirred at 5°C for 30min. The mixture was added to 2M hydrochloric acid (25ml) to give a white precipitate. Water (75ml) was added and the precipitate was collected by filtration to give the title compound as a white solid (456mg, 88%): LCMS retention time 3.54min, *m*/*z* 521 MH⁺.

### Example 40: 6α,9α-Difluoro-11β-hydroxy-16α-methyl-17α-[(3-methylthiophene-2-carbonyl)oxy]-3-oxo-androsta-1,4-diene-17β-carbothioic acid S-fluoromethyl ester

Example 40 was prepared from Intermediate 38 using a method analogous to that described for Example 1: LCMS retention time 3.78min, *m*/*z* 569 MH⁺

### Example 41: 6α,9α-Difluoro-11β-hydroxy-16α-methyl-17α-[(4-methyl-1,3-thiazole-5-carbonyl)oxy]-3-oxo-androsta-1,4-diene-17β-carbothioic acid S-fluoromethyl ester

Example 41 was prepared from Intermediate 39 using a method analogous to that described for Example 1: LCMS retention time 3.51 min, *m*/*z* 570 MH⁺

### Example 42: 17α-[(1-Ethyl-3-methyl-1H-pyrazole-5-carbonyl)oxy]-6α,9α-difluoro-11β-hydroxy-16α-methyl-3-oxo-androsta-1,4-diene-17β-carbothioic acid S-fluoromethyl ester

Example 42 was prepared from Intermediate 40 using a method analogous to that described for Example 1: LCMS retention time 3.64min, *m*/*z* 581 MH⁺

### Example 43: 6α,9α-Difluoro-17α-[(1-methyl-1H-imidazole-5-carbonyl)oxy]-11β-hydroxy-16α-methyl-3-oxo-androsta-1,4-diene-17β-carbothioic acid S-fluoromethyl ester

Example 43 was prepared from Intermediate 41 using a method analogous to that described for Example 1: LCMS retention time 3.14min, *m*/*z* 553 MH⁺

### Example 44: Dry powder composition containing 6α,9α-Difluoro-11β-hydroxy-16α-methyl-17α-[(4-methyl-1,3-thiazole-5-carbonyl)oxy]-3-oxo-androsta-1,4-diene-17β-carbothioic acid S-fluoromethyl ester

A dry powder formulation may be prepared as follows:
6α,9α-Difluoro-11β-hydroxy-16α-methyl-17α-[(4-methyl-1,3-thiazole-5-carbonyl)oxy]-3-oxo-androsta-1,4-diene-17β-carbothioic acid *S*-fluoromethyl ester
   (prepared according to Example 41 and micronised to a MMD of 3µm): 0.20mg
milled lactose (wherein not greater than 85% of particles have a MMD of 60-90µm, and not less than 15% of particles have a MMD of less than 15µm): 12mg
A peelable blister strip containing 60 blisters each filled with a formulation as just described may be prepared.

### Example 45: Dry powder composition containing 6α,9α-Difluoro-11β-hydroxy-16α-methyl-17α-[(4-methyl-1,3-thiazole-5-carbonyl)oxy]-3-oxo-androsta-1,4-diene-17β-carbothioic acid S-fluoromethyl ester and a long acting β₂-adrenoreceptor agonist

A dry powder formulation may be prepared as follows:
6α,9α-Difluoro-11β-hydroxy-16α-methyl-17α-[(4-methyl-1,3-thiazole-5-carbonyl)oxy]-3-oxo-androsta-1,4-diene-17β-carbothioic acid *S*-fluoromethyl ester
(prepared according to Example 41, and micronised to a MMD of 3µm): 0.20mg Long-acting β₂-adrenoreceptor agonist (micronised to a MMD of 3µm):0.02mg milled lactose (wherein not greater than 85% of particles have a MMD of 60-90µm, and not less than 15% of particles have a MMD of less than 15µm): 12mg
A peelable blister strip containing 60 blisters each filled with a formulation as just described may be prepared.

### Example 46: Aerosol formulation containing 6α,9α-Difluoro-11β-hydroxy-16α-methyl-17α-[(4-methyl-1,3-thiazole-5-carbonyl)oxy]-3-oxo-androsta-1,4-diene-17β-carbothioic acid S-fluoromethyl ester

An aluminium canister may be filled with a formulation as follows:
6α,9α-Difluoro-11β-hydroxy-16α-methyl-17α-[(4-methyl-1,3-thiazole-5-carbonyl)oxy]-3-oxo-androsta-1,4-diene-17β-carbothioic acid *S*-fluoromethyl ester
(prepared according to Example 41 and micronised to a MMD of 3µm): 250 µg 1,1,1,2-tetrafluoroethane: to 50µl
(amounts per actuation)
in a total amount suitable for 120 actuations and the canister may be fitted with a metering valve adapted to dispense 50 µl per actuation.

### Example 47: Aerosol formulation containing 6α,9α-Difluoro-11β-hydroxy-16α-methyl-17α-[(4-methyl-1,3-thiazole-5-carbonyl)oxy]-3-oxo-androsta-1,4-diene-17β-carbothioic acid S-fluoromethyl ester and a long acting β₂-adrenoreceptor agonist

An aluminium canister may be filled with a formulation as follows:
6α,9α-Difluoro-11β-hydroxy-16α-methyl-17α-[(4-methyl-1,3-thiazole-5-carbonyl)oxy]-3-oxo-androsta-1,4-diene-17β-carbothioic acid *S*-fluoromethyl ester
(prepared according to Example 41 and micronised to a MMD of 3µm): 250 µg
Long-acting β₂-adrenoreceptor agonist (micronised to a MMD of 3µm): 25 µg 1,1,1,2-tetrafluoroethane: to 50µl
(amounts per actuation)
in a total amount suitable for 120 actuations and the canister may be fitted with a metering valve adapted to dispense 50 µl per actuation.

### Pharmacological Activity

### In Vitro Pharmacological Activity

Pharmacological activity was assessed in a functional *in vitro* assay of glucocorticoid agonist activity which is generally predictive of anti-inflammatory or anti-allergic activity *in vivo.*

The functional assay was based on that described by K.P.Ray et al., Biochem J. (1997), 328, 707-715. A549 cells stably transfected with a reporter gene containing the NF-κB responsive elements from the ELAM gene promoter coupled to sPAP (secreted alkaline phosphatase) were treated with test compounds at appropriate doses for 1 hour at 37°C. The cells were then stimulated with tumour necrosis factor (TNF, 10ng/ml) for 16 hours, at which time the amount of alkaline phosphatase produced is measured by a standard colourimetric assay. Dose response curves were constructed from which EC₅₀ values were estimated.

In this test the compounds of Examples 1 to 9, 11-13, 15-22 and 24-42 showed an EC₅₀ value of <1nM. In this test the compounds of Examples 10, 14 and 23 showed an EC50 value of 1, 2 and 17 nM respectively.

The glucocorticoid receptor (GR) can function in at least two distinct mechanisms, by upregulating gene expression through the direct binding of GR to specific sequences in gene promotors, and by downregulating gene expression that is being driven by other transcription factors (such as NFκB or AP-1) through their direct interaction with GR.

In a variant of the above method, to monitor these functions, two reporter plasmids have been generated and introduced separately into A549 human lung epithelial cells by transfection. The first cell line contains the firefly luciferase reporter gene under the control of a synthetic promoter that specifically responds to activation of the transcription factor NFκB when stimulated with TNFα. The second cell line contains the renilla luciferase reporter gene under the control of a synthetic promotor that comprises 3 copies of the consensus glucocorticoid response element, and which responds to direct stimulation by glucocorticoids. Simultaneous measurement of transactivation and transrepression was conducted by mixing the two cell lines in a 1:1 ratio in 96 well plate (40,000 cells per well) and growing overnight at 37°C. Test compounds were dissolved in DMSO, and added to the cells at a final DMSO concentration of 0.7%. After incubation for 1h 0.5ng/ml TNFα (R&D Systems) was added and after a further 15 hours at 37°C, the levels of firefly and renilla luciferase were measured using the Packard Firelite kit following the manufacturers' directions. Dose response curves were constructed from which EC₅₀ values were determined.

| | Transactivation (GR) | Transrepression (NFκB) |
|---|---|---|
| | ED₅₀(nM) | ED₅₀(nM) |
| Example 1 | 0.06 | 0.20 |
| Metabolite (X) | >250 | >1000 |
| Fluticasone propionate | 0.07 | 0.16 |

### Screen for progesterone receptor activity

The human breast cancer cell line T47D has been reported to upregulate an endogenous alkaline phosphatase in response to progestins (Di Lorenzo et al., Cancer Research (1991) 51, 4470-4475. T47D cells were seeded into 96 well plates at a density of 1x10⁵ cells per well and grown overnight at 37°C. Steroids were dissolved in DMSO, added to the cells (final DMSO concentration 0.7%), and incubated for 24 hours at 37°C. The cells were then washed with PBS and lysed with RIPA buffer (1% IGEPAL, 0.5% Na deoxycholate, 0.1% SDS in phosphate buffered saline). Alkaline phosphatase activity was measured spectrophotometrically (405nm) using p-nitrophenylphosphate (1.5mg/ml) as a substrate dissolved in 1 M diethanolamine, 0.28M NaCl, 0.5mM MgCl₂. Dose response curves were constructed from which EC₅₀ values were estimated.

Examples 12 and 41 were tested for progesterone activity in accordance with the above screen and the selectivity was determined by dividing the ED₅₀ at the progesterone receptor by the ED₅₀ at the glucocorticoid receptor.
The selectivity of Example 12 was determined to be 364
(compare fluticasone propionate: selectivity = 23)
In separate experiment, the selectivity of Example 41 was determined to be 1599
(compare fluticasone propionate: selectivity = 60)

### In Vivo Pharmacological Activity

Pharmacological activity in vivo was assessed in an ovalbumin sensitised Brown Norway rat eosinophilia model. This model is designed to mimic allergen induced lung eosinophilia, a major component of lung inflammation in asthma.

Example 1 produced dose dependant inhibition of lung eosinophilia in this model after dosing as an intra-tracheal (IT) suspension in saline 30 min prior to ovalbumin challenge. Significant inhibition is achieved after a single dose of 30µg of Example 1 and the response was significantly (p=0.016) greater than that seen with an equivalent dose of fluticasone propionate in the same study (69% inhibition with Example 1 vs 41% inhibition with fluticasone propionate).

In a rat model of thymus involution 3 daily IT doses of 100µg of Example 1 induced significantly smaller reductions in thymus weight (p= 0.004) than an equivalent dose of fluticasone propionate in the same study (67% reduction of thymus weight with Example 1 vs 78% reduction with fluticasone propionate).

Taken together these results indicate a superior therapeutic index for Example 1 compared to fluticasone propionate.

### In vitro metabolism in rat and human hepatocytes

Incubation of Example 1 with rat or human hepatocytes shows the compound to be metabolised in an identical manner to fluticasone propionate with the 17-β carboxylic acid (X) being the only significant metabolite produced. Investigation of the rate of appearance of this metabolite on incubation of Example 1 with human hepatocytes (37°C, 10µM drug concentration, hepatocytes from 3 subjects, 0.2 and 0.7 million cells/mL) shows Example 1 to be metabolised ca. 5-fold more rapidly than fluticasone propionate:-

| Subject number | Cell density (million cells/mL) | 17-β acid metabolite production (pmol/h) | |
|---|---|---|---|
| | | Example 1 | Fluticasone propionate |
| 1 | 0.2 | 48.9 | 18.8 |
| 1 | 0.7 | 73.3 | 35.4 |
| 2 | 0.2 | 118 | 9.7 |
| 2 | 0.7 | 903 | 23.7 |
| 3 | 0.2 | 102 | 6.6 |
| 3 | 0.7 | 580 | 23.9 |

Median metabolite production 102-118 pmol/h for Example 1 and 18.8-23.0 pmol/h for fluticasone propionate.

### Pharmacokinetics after intravenous (IV) and oral dosing in rats

Examples 1 and 41 were dosed orally (0.1mg/kg) and IV (0.1 mg/kg) to male Wistar Han rats and pharmacokinetic parameters determined. Example 1 showed negligible oral bioavailability (0.9%) and plasma clearance of 47.3 mL/min/kg, approaching liver blood flow (plasma clearance of fluticasone propionate = 45.2 mL/min/kg).

Examples 4, 19, 24, 25, 28 and 41 were dosed IV (0.1 mg/kg) to male Wistar Han rats and plasma clearance values of 49, 48, 47, 46, 51 and 42 mL/min/kg respectively were determined.

### Pharmacokinetics after intra-tracheal dry powder dosing in the pig.

Anaesthetised pigs (2) were dosed intra-tracheally with a homogenous mixture of Example 1 (1 mg) and fluticasone propionate (1 mg) as a dry powder blend in lactose (10% w/w). Serial blood samples were taken for up to 8h following dosing. Plasma levels of Example 1 and fluticasone propionate were determined following extraction and analysis using LC-MS/MS methodology, the lower limits of quantitation of the methods were 10 and 20pg/mL for Example 1 and fluticasone propionate respectively. Using these methods Example 1 was quantifiable up to 2 hours after dosing and fluticasone propionate was quantifiable up to 8 hours after dosing. Maximum plasma concentrations were observed for both compounds within 15min after dosing. Plasma half-life data obtained from IV dosing (0.1mg/kg) was used to calculate AUC (0-inf) values for Example 1. This compensates for the plasma profile of Example 1 only being defined up to 2 hours after an IT dose and removes any bias due to limited data between Example 1 and fluticasone propionate.

Cₘₐₓ and AUC (0-inf) values show markedly reduced systemic exposure to Example 1 compared to fluticasone propionate:-

| | Cmax (pg/mL) | | AUC (0-inf) (hr.pg/mL) | |
|---|---|---|---|---|
| | Pig 1 | Pig 2 | Pig 1 | Pig 2 |
| Example 1 | 117 | 81 | 254 | 221 |
| Fluticasone propionate | 277 | 218 | 455 | 495 |

The pharmacokinetic parameters for both Example 1 and fluticasone propionate were the same in the anaesthetised pig following intravenous administration of a mixture of the two compounds at 0.1mg/kg. The clearance of these two glucocorticoids is similar is this experimental pig model.

Inhalers of the invention will now be described, by way of example only, with reference to, and as shown in, the accompanying drawings in which:
Figures 1 to 4 relate to a dry powder inhalers;
Figure 5 relates to a metered dose inhaler;
Figure 1 shows a suitable medicament carrier in the form of a capsule.
Figure 2a is a cross-sectional side elevation of a suitable elongate medicament blister strip.
Figure 2b is a top perspective of the medicament blister strip illustrated in Figure 2a.
Figure 3 shows a cross-sectional dry powder inhaler (DPI) comprising a powder reservoir.
Figure 4 shows a cross-sectional dry powder inhaler (DPI) comprising an elongate medicament blister strip.

The medicament carrier in Figure 1 is in the form of a capsule 1 comprising a wall 2 enclosing medicament powder 5. Medicament powder 5 is released on piercing the wall 2 of capsule 1 and may be inhaled by a patient.

Figure 2a shows a sectional side-elevation of a single blister strip 106 comprising a pocket 107, containing dry powder 105, base 110 and lid comprising laminates 114, 115. The lid is composed of a metallic foil laminate 114 bound to a plastic laminate 115. In the diagram, the lid 114, 115 is hermetically sealed to base 110 by appropriate means (e.g. adhesion, welding). Base 110 comprises an organic polymeric plastic 103. A top perspective view of the blister strip 106 showing pockets 107 is illustrated in Figure 2b. Laminated lid 114, 115 is sealed to base 110.

Figure 3 shows a sectional view of a dry powder inhaler 420 for dispensing medicament in accord with the present invention. The inhaler 420 comprises a body 421 which defines a reservoir 423 and a reservoir cover 424. The reservoir contains a supply of medicament in dry powder form 405. The walls 423 of the reservoir, defined by the body 421, are comprised of a plastic material 403. Base 425 and body 421 define an aperture 430 through which powder 405 can pass from the reservoir to the dosing member 432. Powder 405 is guided by the walls 423 of the reservoir, which form a hopper, to the dosing member 432. Extending laterally from the lower end of the main body 421 is mouthpiece 435, through which the patient inhales via passage 433. If the device were intended for nasal inhalation this would be replaced by a nosepiece.

Figure 4 shows a simplified cross-sectional plan view of a dry powder inhaler comprising an elongate medicament carrier suitable for dispensing medicament in accord with the present invention. The inhaler 540 dispenses unit doses of medicament powder from a medicament blister strip 506. The inhaler is comprised of an outer casing 544 enclosing a medicament strip 506 within body 521. The medicament strip may be, for example, any of those described in Figures 2a to 2b above. The patient uses the inhaler by holding the device to his mouth, depressing lever 538, and inhaling through mouthpiece 535. Depression of lever 538 activates the internal mechanism of the inhaler, such that the lid 514 and base 510 sheets of coiled medicament blister strip 506 are separated at index wheel 541 by use of contracting wheel 542 and base wheel 543. A unit dose of powdered medicament within blister pocket 507 is released and may be inhaled by the patient through exit port 533 and mouthpiece 535.

Figure 5 is a schematic representation of a section through a standard metered dose inhalation device.

The standard metered dose inhaler shown in Fig 1 comprises a housing 10 in which an aerosol canister 20 can be located. The housing is open at one end (which will hereinafter be considered to be the top of the device for convenience of description) and is closed at the other. An outlet 30 leads laterally from the closed end of the housing 10. In the embodiment illustrated, the outlet 30 is in the form of a mouthpiece intended for insertion into the mouth of the patient but it may, if desired, be designed as a nozzle for insertion into the patient's nostril.

The aerosol canister 20, comprising a neck region 21 and ferrule 22, has an outlet valve stem 40 at one end. This valve member can be depressed to release a measured dose from the aerosol canister or, alternatively, the valve stem 40 can be fixed and the main body of the canister can be moved relative to the valve member to release the dose.

As shown in Figure 5, the aerosol canister 20 is located in the housing 10 so that one end protrudes from its open top, the canister being positioned such that the neck 21 and valve ferrule 22 are enclosed within housing 10. Spacer ribs (not shown) may be provided inside the housing to hold the external surface of the canister 20 spaced from the internal surface of the housing 10. A support 50 is provided at the lower end of the housing 10 and has a passage 60 in which the valve stem 40 of the aerosol canister 20 can be located and supported. A second passage 70 is provided in the support 50 and is directed towards the interior of the outlet 30. Thus, when the parts are in the positions shown in Figure 1, the protruding portion of the aerosol canister 20 can be depressed to move the canister relative to the valve stem 40 to open the valve and a dose of medicament contained in the aerosol will be discharged through the passage 70 and into the outlet 30 from which it can be inhaled by a patient. One dose will be released from the aerosol canister each time it is fully depressed.

The body 6 is made from a plastic material and defines a housing 9 and a dispensing nozzle 11. The housing 9 defines a cavity formed by a side wall and a first end wall and a second end wall 14. The dispensing nozzle 11 is connected to and extends away from the second end wall 14 and has an external tapering form.

Throughout the specification and the claims which follow, unless the context requires otherwise, the word 'comprise', and variations such as 'comprises' and 'comprising', will be understood to imply the inclusion of a stated integer or step or group of integers but not to the exclusion of any other integer or step or group of integers or steps.

The patents and patent applications described in this application are herein incorporated by reference.

## Claims

1. A pharmaceutical formulation for administration by inhalation comprising a compound of formula (I), 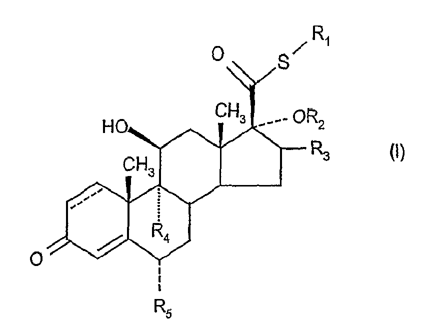 wherein
R₁ represents C₁₋₆ alkyl or C₁₋₆ haloalkyl;
R₂ represents -C(=O)-aryl or -C(=O)-heteroaryl;
R₃ represents hydrogen, methyl (which may be in either the α or β configuration) or methylene;
R₄ and R₅ are the same or different and each represents hydrogen or halogen; and
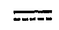 represents a single or a double bond;
or a salt or solvate thereof, together with a long-acting β₂-adrenoreceptor agonist which formulation has a therapeutically useful effect in the treatment of inflammatory disorders of the respiratory tract over a period of 24 hours or more.

2. A pharmaceutical formulation according to claim 1 wherein the compound of formula (I) or a solvate thereof and the long-acting β₂-adrenoreceptor agonist are both present in particulate form.

3. A pharmaceutical formulation according to claim 2 or claim 3 wherein the formulation further comprises a particulate carrier.

4. A pharmaceutical formulation according to claim 3 wherein the carrier is lactose.

5. A pharmaceutical formulation according to any one of the preceding claims further comprising a liquified propellant gas.

6. A pharmaceutical formulation according to any one of the preceding claims wherein the inflammatory disorder of the respiratory tract is asthma.

7. A pharmaceutical formulation according to any one of the preceding claims wherein the long-acting β₂-adrenoreceptor agonist is a compound of formulas (M): 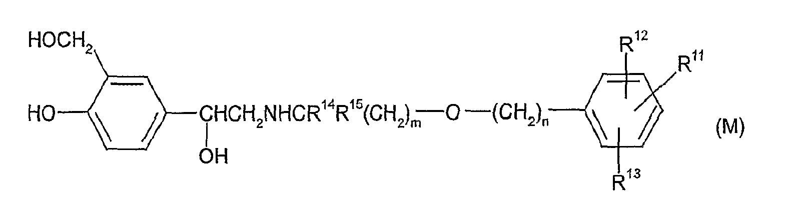 or a salt or solvate thereof, wherein:
m is an integer of from 2 to 8;
n is an integer of from 3 to 11,
with the proviso that m + n is 5 to 19,
R¹¹ is -XSO₂NR¹⁶R¹⁷ wherein X is -(CH₂)ₚ- or C₂₋₆ alkenylene;
R¹⁶ and R¹⁷ are independently selected from hydrogen, C₁₋₆alkyl, C₃₋₇cycloalkyl, C(O)NR¹⁸R¹⁹, phenyl, and phenyl (C₁₋₄alkyl)-,
or R¹⁶ and R¹⁷, together with the nitrogen to which they are bonded, form a 5-, 6-, or 7- membered nitrogen containing ring, and R¹⁶ and R¹⁷ are each optionally substituted by one or two groups selected from halo, C₁₋₆alkyl, C₁₋₆haloalkyl, C₁₋₆alkoxy, hydroxy-substituted C₁₋₆alkoxy, -CO₂R¹⁸,-SO₂NR¹⁸R¹⁹, -CONR¹⁸R¹⁹, -NR¹⁸C(O)R¹⁹, or a 5-, 6- or 7-membered heterocylic ring;
R¹⁸ and R¹⁹ are independently selected from hydrogen, C₁₋₆alkyl, C₃₋₆cycloalkyl, phenyl, and phenyl (C₁₋₄alkyl)-; and
p is an integer of from 0 to 6, preferably from 0 to 4;
R¹² and R¹³ are independently selected from hydrogen, C₁₋₆alkyl, C₁₋₆alkoxy, halo, phenyl, and C₁₋₆haloalkyl; and
R¹⁴ and R¹⁵ are independently selected from hydrogen and C₁₋₄alkyl with the proviso that the total number of carbon atoms in R¹⁴ and R¹⁵ is not more than 4.

8. A pharmaceutical formulation according to any one of the proceding claims wherein the compound of formula (I) is 6α,9α-Difluoro-11β-hydroxy-16α-methyl-17α-[(4-methyl-1,3-thiazole-5-carbonyl)oxy]-3-oxo-androsta-1,4-diene-17β-carbothioic acid *S*-fluoromethyl ester or a salt or solvate thereof.

9. A method of treatment of a inflammatory disorder of the respiratory tract once-per-day which comprises administration of a pharmaceutical formulation according to any one of claims 1 to 8.

10. A method of treatment according to claim 9 wherein the inflammatory disorder of the respiratory tract is asthma.

11. A method according to claim 8 or claim 9 wherein the compound of formula (I) is 6α,9α-Diftuoro-11β-hydroxy-16α-methyl-17α-[(4-methyl-1,3-thiazole-5-carbonyl)oxy]-3-oxo-androsta-1,4-diene-17β-carbothioic acid *S*-fluoromethyl ester or a salt or solvate thereof.

12. An inhaler containing a plurality of doses of a pharmaceutical formulation comprising a compound of formula (I) 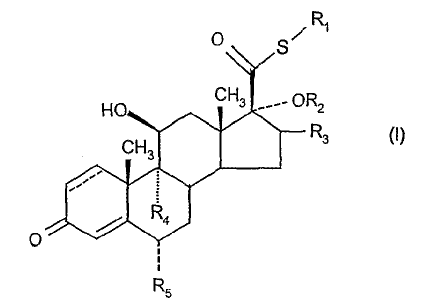 wherein
R₁ represents C₁₋₆ alkyl or C₁₋₆ haloalkyl;
R₂ represents -C(=O)-aryl or -C(=O)-heteroaryl;
R₃ represents hydrogen, methyl (which may be in either the α or β configuration) or methylene;
R₄ and R₅ are the same or different and each represents hydrogen or halogen; and
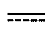 represents a single or a double bond;
or a salt or solvate thereof, together with a long-acting β₂-adrenoreceptor agonist which formulation has a therapeutically useful effect in the treatment of inflammatory disorders of the respiratory tract over a period of 24 hours or more, and which doses are suitable for once-per-day administration of the formulation by inhalation.

13. An inhaler according to claim 12 wherein the compound of formula (I) or a solvate thereof and the long-acting β₂-adrenoreceptor agonist are both present in particulate form.

14. An inhaler according to claim 12 or 13 wherein the formulation further comprises a particulate carrier.

15. An inhaler according to claim 14 wherein the carrier is lactose.

16. An inhaler according to any one of claims 12 to 15 wherein the formulation further comprises a liquefied propellant gas.

17. An inhaler containing a plurality of doses of a pharmaceutical formulation comprising a particulate compound of formula (I) 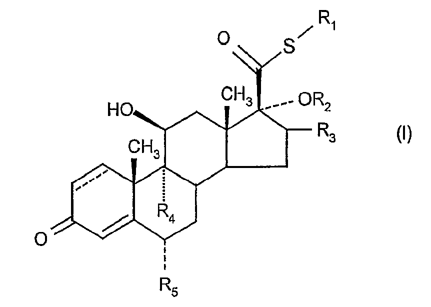 wherein
R₁ represents C₁₋₆ alkyl or C₁₋₆ haloalkyl;
R₂ represents -C(=O)-aryl or -C(=O)-heteroaryl;
R₃ represents hydrogen, methyl (which may be in either the α or β configuration) or methylene;
R₄ and R₅ are the same or different and each represents hydrogen or halogen; and
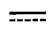 represents a single or a double bond;
or a salt or solvate thereof, together with a long-acting β₂-adrenoreceptor, a particulate long-acting β₂-adrenoreceptor agonist and a carrier each drug being present in an amount adequate to provide a therapeutically useful effect in the treatment of inflammatory disorders of the respiratory tract over a period of 24 hours or more following once-per-day dosing by inhalation.

18. An inhaler according to any one of claims 12 to 17 wherein the inflammatory disorder of the respiratory tract is asthma.

19. An inhaler according to any one of claims 12 to 18 wherein the long-acting β₂-adrenoreceptor agonist is a compound of formula (M): 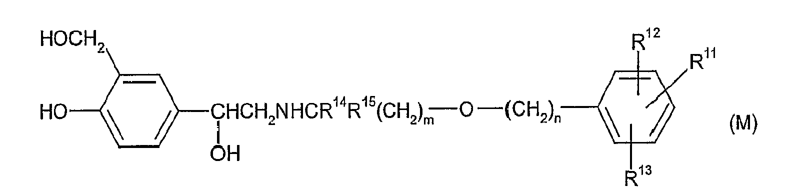 or a salt or solvate thereof, wherein:
m is an integer of from 2 to 8;
n is an integer of from 3 to 11,
with the proviso that m + n is 5 to 19,
R¹¹ is -XSO₂NR¹⁶R¹⁷ wherein X is -(CH₂)ₚ- or C₂₋₆ alkenylene;
R¹⁶ and R¹⁷ are independently selected from hydrogen, C₁₋₆alkyl, C₃₋₇cycloalkyl, C(O)NR¹⁸R¹⁹, phenyl, and phenyl (C₁₋₄alkyl)-,
or R¹⁶ and R¹⁷, together with the nitrogen to which they are bonded, form a 5-, 6-, or 7- membered nitrogen containing ring, and R¹⁶ and R¹⁷ are each optionally substituted by one or two groups selected from halo, C₁₋₆alkyl, C₁₋₆haloalkyl, C₁₋₆alkoxy, hydroxy-substituted C₁₋₆alkoxy, -CO₂R¹⁸,-SO₂NR¹⁸R¹⁹, -CONR¹⁸R¹⁹, -NR¹⁸C(O)R¹⁹, or a 5-, 6- or 7-membered heterocylic ring;
R¹⁸ and R¹⁹ are independently selected from hydrogen, C₁₋₆alkyl, C₃₋₆cycloalkyl, phenyl, and phenyl (C₁₋₄alkyl)-; and
p is an integer of from 0 to 6, preferably from 0 to 4;
R¹² and R¹³ are independently selected from hydrogen, C₁₋₆alkyl, C₁₋₆alkoxy, halo, phenyl, and C₁₋₆haloalkyl; and
R¹⁴ and R¹⁵ are independently selected from hydrogen and C₁₋₄alkyl with the proviso that the total number of carbon atoms in R¹⁴ and R¹⁵ is not more than 4.

20. An inhaler according to any one of claims 12 to 19 wherein the compound of formula (I) is 6α,9α-Difluoro-11β-hydroxy-16α-methyl-17α-[(4-methyl-1,3-thiazole-5-carbonyl)oxy]-3-oxo-androsta-1,4-diene-17β-carbothioic acid *S-*fluoromethyl ester or a salt or solvate thereof.

21. A formulation according to any one of claims 1 to 8 for use in human or veterinary medicine in the treatment of patients with inflammatory and/or allergic condition, for treatment once per-day

22. The use of a formulation as according to any one of claims 1 to 8 for the manufacture of a medicament for the treatment of patient with inflammatory and/or allergic conditions for treatment once-per-day.

23. A method for the treatment of a human or animal subject with an anti-inflammatory and/or allergic condition, which method comprises administering to said human or animal subject an effective amount of a formulation as claimed in any one of claims 1 to 8.
